(19) Europäisches Patentamt
European Patent Office
Office européen des brevets

(11) Publication number: **0 061 172**
**B1**

(12) # EUROPEAN PATENT SPECIFICATION

(45) Date of publication of patent specification: **19.06.85**

(51) Int. Cl.$^4$: **C 07 F 9/38,** C 12 P 21/02, C 12 R 1/03, C 12 R 1/04, A 61 K 37/02

(21) Application number: **82102277.9**

(22) Date of filing: **19.03.82**

(54) **Phosphorus-containing oligopeptides, processes for preparation thereof and a pharmaceutical composition containing the same.**

(30) Priority: **20.03.81 JP 40651/81**
**19.08.81 JP 128740/81**
**17.11.81 JP 184198/81**
**17.11.81 JP 184199/81**

(43) Date of publication of application:
**29.09.82 Bulletin 82/39**

(45) Publication of the grant of the patent:
**19.06.85 Bulletin 85/25**

(84) Designated Contracting States:
**DE FR GB IT**

(56) References cited:
**EP-A-0 002 822**
**AT-A- 342 192**

(73) Proprietor: **KYOWA HAKKO KOGYO CO., LTD.**
**Ohtemachi Bldg., 6-1 Ohtemachi I-chome**
**Chiyoda-ku Tokyo 100 (JP)**

(72) Inventor: **Kase, Hiroshi**
**3-35-18 Maehara-cho**
**Koganei-shi Tokyo (JP)**
Inventor: **Yamato, Masayuki**
**3-9-11, Naka-machi**
**Machida-shi Tokyo (JP)**
Inventor: **Koguchi, Toshiro**
**6715-6, Motoishikawa-cho Midori-ku**
**Yokohama-shi Kanagawa-ken (JP)**
Inventor: **Okachi, Ryo**
**1188, Shimotogari, Nagaizumi-cho**
**Sunto-gun Shizuoka-ken (JP)**
Inventor: **Kasai, Masaji**
**206, Congressional Lane**
**Rockville Maryland 20852 (US)**
Inventor: **Shirahata, Kunikatsu**
**4-11-5, Iwatominami**
**Komae-shi Tokyo (JP)**
Inventor: **Kawamoto, Isao**
**1-21-2, Fujimino**
**Hiratsuka-shi Kanagawa-ken (JP)**
Inventor: **Shuto, Katsuichi**
**410-1, Nameri, Nagaizumi-cho**
**Sunto-gun Shizuoka-ken (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 061 172 B1

Inventor: **Karasawa, Akira**
**411-3, Shimonagakubo Nagaizumi-cho**
**Sunto-gun Shizyoka-ken (JP)**
Inventor: **Deguchi, Takashi**
**734-17, Tokura**
**Mishima-shi Shizuoka-ken (JP)**
Inventor: **Nakayama, Kiyoshi**
**5-16-9, Minamidai**
**Sagamihara-shi Kanagawa-ken (JP)**

(74) Representative: **Casalonga, Axel et al**
**BUREAU D.A. CASALONGA OFFICE JOSSE & PETIT Baaderstrasse 12-14**
**D-8000 München 5 (DE)**

**Description**

*Summary of the Invention*

The present invention relates to phosphorus-containing oligopeptides, processes for preparation thereof and a pharmaceutical composition containing the same.

The oligopeptides are novel compounds which have the structure shown hereinafter and hypotensive activity.

The oligopeptides are prepared by organic synthesis and some of them also by cultivation of a certain microorganism.

*Brief Description of Drawings*

Fig. 1 is ultraviolet absorption spectrum of K—26 hereinafter defined, where (1) shows results of measurement in a neutral state, (2) in an acid state, and (3) in an alkaline state.

Fig. 2 is infrared absorption spectrum of K—26.

Fig. 3 is PMR spectrum of K—26 (measured in heavy water).

Fig. 4 is CMR spectrum of K—26.

Fig. 5 is ultraviolet absorption spectrum of K—4 hereinafter defined, where curve (1) shows spectrum of K—4 in 0.1N aqueous sodium hydroxide and curve (2) spectrum in 0.1N hydrochloric acid (concentration: 200 µg/ml).

Fig. 6 is ultraviolet absorption spectrum of K—4.

Fig. 7 is PMR spectrum of K—4.

Fig. 8 is $^{13}$C-NMR spectrum of K—4.

*Detailed Explanation of the Invention*

The phosphorus-containing oligopeptides of the present invention having a hypotensive activity are represented by the following formula [I]:

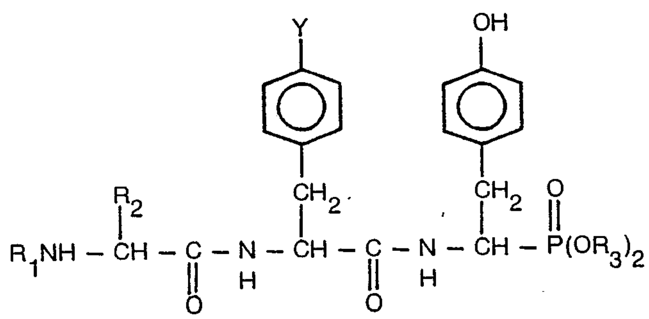

[I]

[wherein $R_1$ represents hydrogen, lower alkyl, substituted or unsubstituted phenylalkyl, or $R_4CO$— (where $R_4$ represents hydrogen, lower alkyl, or substituted or unsubstituted phenylalkyl); $R_2$ represents lower alkyl; $R_3$ represents hydrogen, lower alkyl, or substituted or unsubstituted phenylalkyl; and Y represents hydrogen or hydroxyl] (hereinafter referred to as Compound [I]; Compounds of other formulae are similarly designated).

The term "lower alkyl" in the definition of $R_1$, $R_2$, $R_3$ and $R_4$ in the formula [I] means straight or branched alkyl having 1—6 carbon atoms.

The term "alkyl" in "substituted or unsubstituted phenylalkyl" in the definition of $R_1$, $R_3$ and $R_4$ means straight or branched alkyl having 1—6 carbon atoms, and the substituent are halo such as chloro and bromo, nitro or alkoxy having 1—3 carbon atoms such as methoxy and ethoxy. Halo or alkoxy substituted at the para-position is especially preferable as the substituent.

Compound [I] includes all of the optical isomers. Most preferable Compounds [I] are those where the configuration of the radicals derived from the two amino acid are L-form and the radical derived from the 1-amino-2-(4-hydroxyphenyl)ethylphosphonic acid has a negative specific rotation.

Among Compounds [I], N-(N-acyl-L-isoleucyl-L-tyrosyl)-(−)-1-amino-2-(4-hydroxyphenyl)ethylphosphonic acid ester represented by the formula [I—1]:

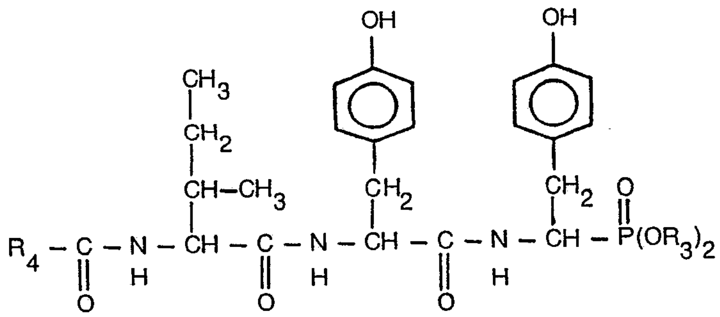

[I—1]

(wherein $R_3$ and $R_4$ have the same meaning as defined above) and N-(N-substituted-L-valyl-L-phenylalanyl)-(−)-1-amino-2-(4-hydroxyphenyl)ethylphosphonic acid ester represented by the formula [I—2]:

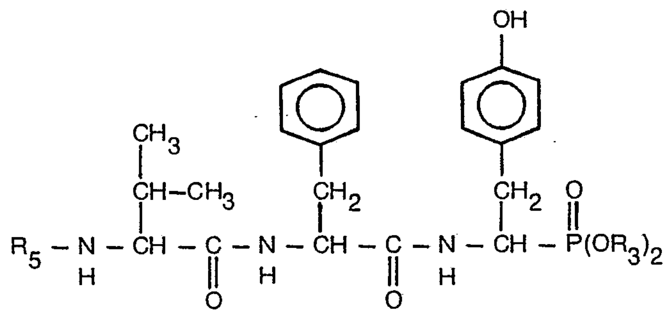

[I—2]

(wherein $R_5$ has the same meaning as $R_1$ excluding

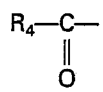

and $R_3$ has the same meaning as defined above) are typical examples of the compounds according to the present invention.

Compound [I] is synthesized through the following steps:

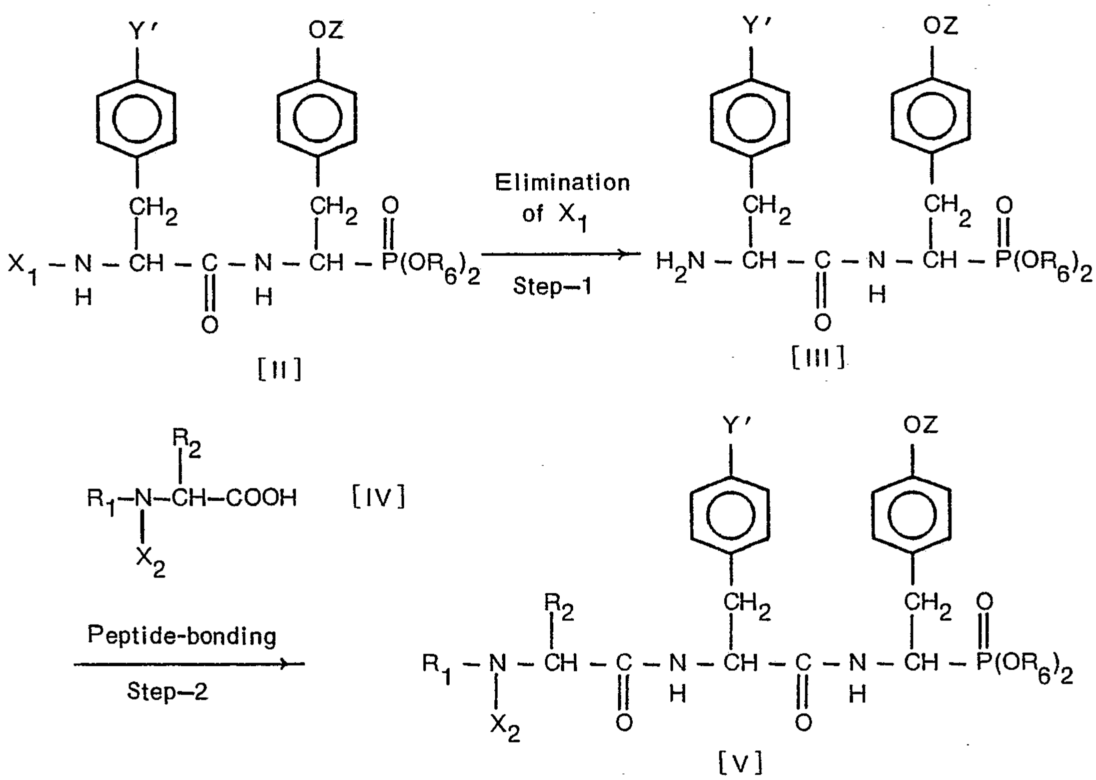


[II] [III] [IV] [V]

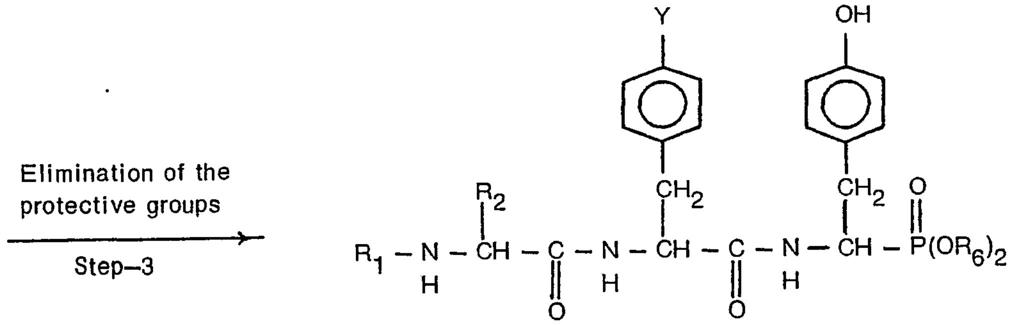


[I—A]

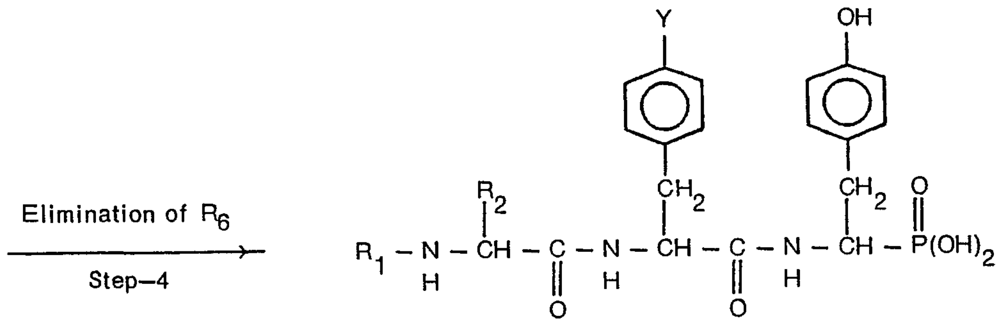


[I—B]

In the above formulae, definition of each symbol is as follows:

$X_1$: an amino-protecting group usually used in the peptide synthesis such as benzyloxycarbonyl and t-butoxycarbonyl
$X_2$: hydrogen when $R_1$ is $R_4CO$—, or same as $X_1$ when $R_1$ is hydrogen, alkyl or substituted or unsubstituted phenylalkyl
Y′: hydrogen, hydroxyl or OZ
Z: a hydroxyl-protecting group usually used in the peptide synthesis such as benzyl
$R_6$: $R_3$ excluding hydrogen.

Compound [I] consists of Compound [I—A] and Compound [I—B].
Each step is described in detail below.

*Step—1:*
Compound [III] can be obtained from Compound [II] by removing an amino-protecting group by methods usually used in peptide synthesis.

For example, when $X_1$ is a benzyloxycarbonyl group, the desired compound can be obtained by catalytic reduction. In the case of t-butoxycarbonyl group, the desired compound can be obtained by mild hydrolysis with an acid.

Compound [III] is a novel compound and is prepared by the method hereinafter described.

*Step—2:*
Compound [V] can be obtained by reacting Compound [III] with an amino acid derivative represented by the formula [IV]:

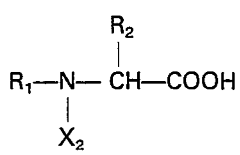

[IV]

(wherein $R_1$, $R_2$ and $X_2$ have the same meaning as defined above) in the presence of a condensing agent usually used in the peptide synthesis, and separating and purifying the product according to methods usually used in organic synthesis. Compound [V] can also be obtained by converting said amino acid derivative to an active ester derivative usually used in the peptide synthesis, condensing Compound [III] therewith, and then separating the product.

As the condensing agent, N,N′-dicyclo-hexylcarbodiimide (hereinafter referred to as DCC), DCC and N-hydroxysuccinimide and DCC and 1-oxybenzotriazole, can be used. The reaction solvent is a solvent usually used in the peptide synthesis, and for example, ethers such as tetrahydrofuran or ethylene glycol dimethyl ether, amides such as N,N-dimethylformamide, halogenated hydrocarbons such as methylene chloride, or their mixtures can be used. As the active ester, substituted phenyl ester such as p-nitrophenyl ester of said amino acid derivative, or dicarboxyl acid imide esters such as N-hydroxysuccinimide ester, or acid anhydrides of said amino acid derivative can be used.

*Step—3:*

Compound [I—A] can be obtained from Compound [V] according by removing the amino- or hydroxyl-protecting group by methods usually used in peptide synthesis. For example, when $X_2$ is a benzyloxycarbonyl group, the desired compound can be obtained by catalytic reduction. In the case of t-butoxycarbonyl group, the desired compound can be obtained by mild hydrolysis with an acid. When Z is a benzyloxycarbonyl group or a benzyl group, the desired compound can be obtained in the same manner as in the case of said $X_2$.

*Step—4:*

Compound [I—B] can be obtained by hydrolyzing the phosphoric acid ester of Compound [I—A]. For example, Compound [I—B] can be obtained by hydrolysis of Compound [I—A] in an acetic acid solution containing hydrogen chloride or hydrogen bromide.

Compound [II] can be obtained by the following steps:

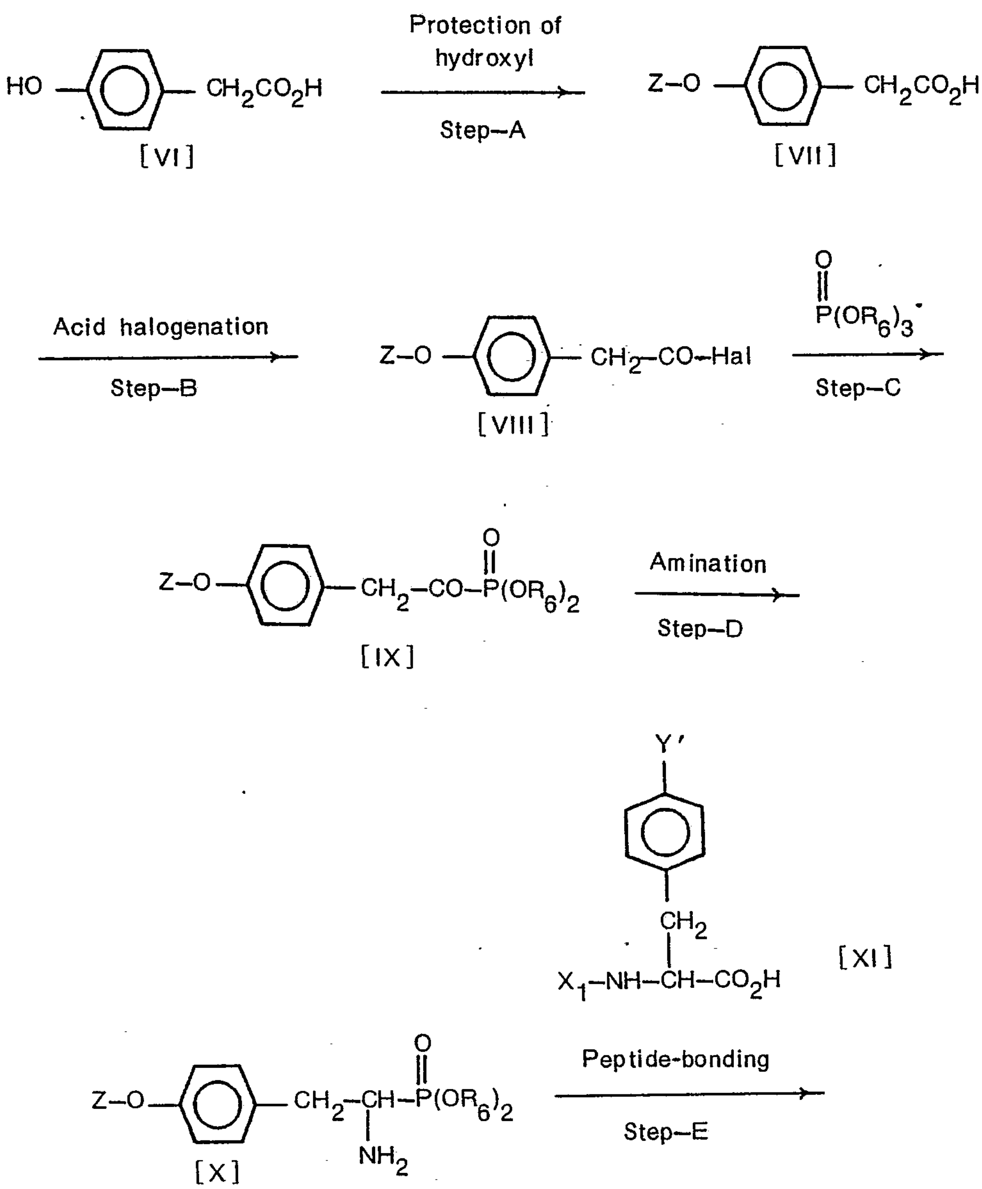

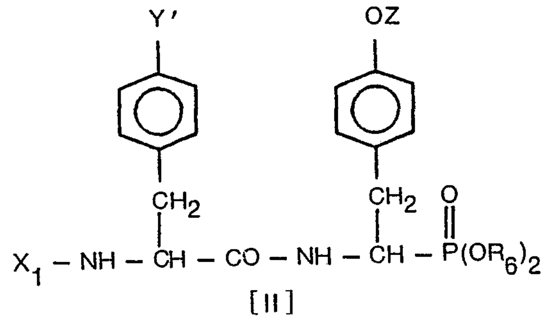

[II]

In the above, Hal means halogen such as chlorine, bromine and iodine.

The respective steps will be described in detail below:

*Step—A:*

Compound [VII] can be obtained by reaction of Compound [VI] with alkyl halide, arylalkyl halide or benzyloxycarbonyl halide, in an aqueous solution of sodium or potassium hydroxide, or an alkali metal carbonate. As the alkyl halide, methyl iodide or ethyl iodide, and as the arylalkyl halide, benzyl bromide, or benzyl chloride, are used. The reaction is carried out at room temperature for 2—24 hours.

*Step—B:*

Compound [VIII] can be obtained from Compound [VII] by methods usually used in organic synthesis for preparing an acid halide from carboxylic acid. For example, Compound [VII] is dissolved in thionyl chloride, and refluxed for 1—12 hours, whereby Compound [VIII] is obtained.

*Step—C:*

Compound [IX] can be obtained by reacting Compound [VIII] with phosphite ester represented by the formula $P(OR_6)_3$ wherein $R_6$ has the same meaning as defined above in an inert solvent with stirring at room temperature or at an elevated temperature for 30 minutes to 3 days, preferably in nitrogen gas stream.

*Step—D*

Compound [X] can be obtained from compound [IX] by methods usually used in organic synthesis for converting a ketone to an amino group. For example, Compound [IX] is reacted with hydroxylamine or dialkylhydrazine at 10—60°C for 0.5—12 hours to give the corresponding oxime or dialkylhydrazone, which is subjected to catalytic reduction using a catalyst, or to reduction using a reducing agent such as aluminium amalgum or zinc-acetic acid (0—100°C for 1—24 hours), whereby Compound [X] is obtained.

*Step—E:*

Compound [II] can be obtained by reacting Compound [X] with an amino acid derivative represented by the formula [XI]:

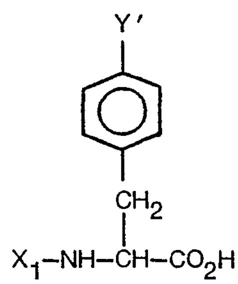

[XI]

(wherein $X_1$ and Y' have the same meaning as defined above) in the presence of a condensing agent usually used in the peptide synthesis in an inert solvent at −20°C to room temperature for a few hours to 3 days, and by separating and purifying the reaction product by methods usually used in organic synthesis. Alternatively, the amino acid derivative is converted to an active ester derivative usually used in peptide synthesis, and then the latter derivative is condensed with compound (VII), and thereafter compound (II) can be obtained by separation.

As the condensing agent and the inert solvent, those enumerated in the aforesaid Step—2 are usable. As the active ester, substituted phenyl esters such as p-nitrophenyl ester, dicarboxylic acid imide esters such as N-hydroxysuccinimide ester or acid anhydrides of the amino acid derivative can be used.

When an amino acid derivative of the formula [XI] having L-configuration is used, Compound [II] where 1-amino-2-(4-hydroxyphenyl)ethylphosphonic acid part has a negative specific rotation (hereinafter

referred to as L-(−)-Compound [II]) is obtained by separation of diastereomers after completion of the reaction.

L-(−)-Compound [II] or a compound obtained by eliminating the protective group Z of L-(−)-Compound [II] according to a conventional elimination method is hydrolyzed under such condition that the asymmetry of 1-amino-2-(4-hydroxyphenyl)ethylphosphonic acid is maintained to produce (−)-1-amino-2-(4-substituted or unsubstituted hydroxy phenyl) ethylphosphonic acid represented by the formula [XII]:

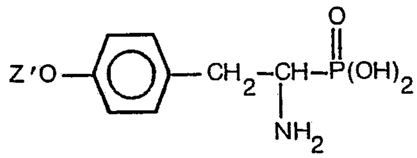

[XII]

(wherein Z′ represents hydrogen or Z). For example, it can be obtained with heating in an aqueous solution of mineral acid such as hydrochloric acid at 100—120°C for half a day to two days and by a separation and purification means used in ordinary organic synthesis.

(−)-Compound [XII] is either treated with etherial diazomethane at room temperature for 5 minutes to 3 hours, or reacted with a lower alcohol such as methanol, ethanol, propanol and butanol in the presence of DCC in a mixed solvent of dimethylformamide and pyridine (2:1) at room temperature overnight to produce an ester represented by the formula [XIII]

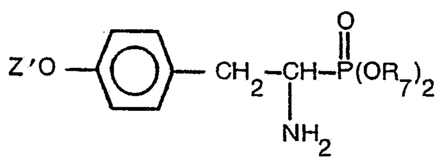

[XIII]

(wherein $R_7$ is lower alkyl and Z′ has the same meaning as defined above). (−)-Compound [XIII] can also be obtained according to a method illustrated in Reference example 5.

(−)-Compound [II] or (−)-Compound [I] can be obtained by conducting the reactions of Step—E and later steps using (−)-Compound [XIII] in place of Compound [X].

Among Compounds [I], N-(N-acetyl-L-isoleucyl-L-tyrosyl)-(−)-1-amino-2-(4-hydroxyphenyl)ethylphosphonic acid represented by the formula:

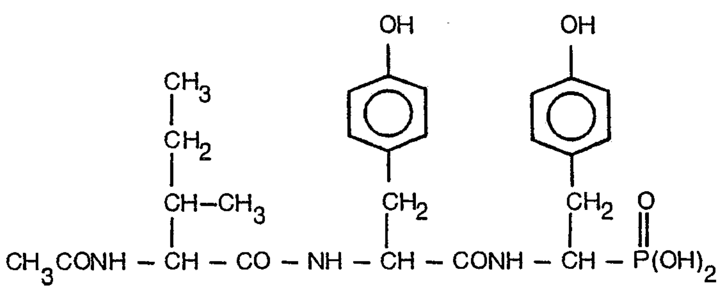

(hereinafter referred to as K—26) and N-(N-methyl-L-valyl-L-phenylalanyl)-(−)-1-amino-2-(4-hydroxyphenyl)ethylphosphonic acid represented by the formula:

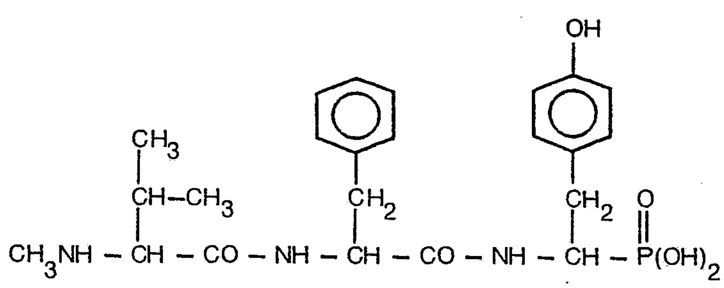

(hereinafter referred to as K—4) are can also be obtained by cultivating a specific microorganism.

For the production of K—26, microorganisms belonging to *Actinomycetes* are used. An example of suitable strains is a strain K—26 isolated by the present inventors from a soil sample from the bank at Kadoike in Numazu City in Shizuoka Prefecture. Morphological, cultural and physiological characteristics of

the strain determined according to the procedure described in detail by Searing, et al in Intern. J. Syst. Bacteriol. *16* 313—340 (1966) are described below:

I. Growth on various culture media

Growth and color characteristics when cultivated on various culture media at 28°C for 2 weeks are given below. Color identification is made in accordance with the color classification of "Color Harmony Manual (Container Corporation of America)".

1) Sucrose-nitrate agar medium
Growth: very poor

2) Glucose-asparagine agar medium
Growth: very poor

3) Glycerol-asparagine agar medium (ISP No. 5)
Growth: very poor

4) Glycerol-calcium malate agar medium
Growth: very poor

5) Egg-albumin agar medium
Growth: very poor

6) Starch-inorganic salt agar medium (ISP No. 4)
Growth: poor, smooth
Reverse color: bright coral red (6 na)
Aerial mycelium: none
Soluble pigment: none

7) Nutrient agar medium
Growth: poor or moderate, granular
Reverse color: bright shell pink (6 ea) to bright peach (5 ia)
Aerial mycelium: none
Soluble pigment: none

8) Yeast extract-malt extract agar medium (ISP No. 2)
Growth: poor or moderate, granular
Reverse color: bright coral red (6 na) to tomato red (6½ pe)
Aerial mycelium: none
Soluble pigment: none

9) Oatmeal agar medium (ISP No. 3)
Growth: good, smooth
Reverse color: chinese red (6 pc)
Aerial mycelium: none
Soluble pigment: none

10) Glucose-yeast extract agar medium
Growth: moderate or good, granular
Reverse color: bright coral red (6 pa) to tomato red (6½ pa)
Aerial mycelium: none
Soluble pigment: none

11) Bennet's agar medium
Growth: moderate, granular
Reverse color: apricot (4 ga) to chinese red (6 pd)
Aerial mycelium: poor, white (a)
Soluble pigment: none

12) Emerson's agar medium:
Growth: poor or moderate
Reverse color: bright coral red (6 pa)
Aerial mycelium: none
Soluble pigment: none

13) Hickey-Tresner's agar medium

| | |
|---|---|
| Growth: | moderate, granular |
| Reverse color: | light cherry red (6 la) to chinese red (6 pc) |
| Aerial mycelium: | poor, white (a) |
| Soluble pigment: | none |

14) Peptone-yeast extract-iron agar medium (ISP No. 6)

| | |
|---|---|
| Growth: | poor, smooth |
| Reverse color: | peach (5 ga) |
| Aerial mycelium: | none |
| Soluble pigment: | none |

15) Tyrosine agar medium (ISP No. 7)

| | |
|---|---|
| Growth: | poor, smooth |
| Reverse color: | fresh pink (5 ca) to bright orange (5 na) |

II. Physiological properties

Physiological properties of strain K—26 are given below.

In the tests except those on the optimum temperature and actions upon gelatin, milk and cellulose, the strain is cultivated at 28°C for 2 weeks. The temperature is determined after 5 days of cultivation and actions upon milk, gelatin and cellulose are observed after cultivation at 28°C for 4 weeks. The present strain fails to grow on the inorganic medium (ISP No. 9) of Pridham-Gottlieb, and thus the test of utilization of a carbon source is carried out on the Ludemann's medium (N.Y. Acad. Sci. *33* 207, 1971).

1) Utilization of carbon source: D-xylose, D-glucose, D-fructose, D-galactose and starch are utilized. D-arabinose, inositol, L-rhamnose, glycerol, D-lactose and α-melibiose are not utilized. Raffinose, mannitol and mannose are slightly utilized.

| | |
|---|---|
| 2) Liquefaction of gelatin: | negative |
| 3) Action upon milk: | neither coagulation nor liquefaction |
| 4) Decomposition of cellulose: | negative |
| 5) Hydrolysis of starch: | positive |
| 6) Optimum growth pH: | 6.5—7.8 |
| 7) Optimum growth temperature: | 28°C—37°C |
| 8) Formation of tyrosinase: | negative |
| 9) Formation of melanoid pigment: | negative |

III. Cell wall composition

As amino acids in the cell wall, *meso*-diaminopimelic acid, alanine and glutamic acid are contained, but neither LL-diaminopimelic acid nor glycine is contained. By analysis of sugars in the whole-cell according to the method of Lechevalier et al (The Actinomycetales, Gustav Fister Verlag. Jena. 311—316, 1970), ribose, glucose, galactose, madurose and a small amount of mannose are detected, but neither arabinose or xylose is detected. According to the Lechevalier et al classification (Intern. J. System. Bacteriol. *20* 435—443, 1970), strain K—26 has cell walls of type III and a type B whole-cell sugar pattern.

IV. Morphological properties

The present strain grows poorly on a chemically defined medium such as sucrose-nitrate agar medium, glucose-asparagine agar medium, etc., but grows vigorously on a natural nutrient medium such as glucose-yeast extract agar medium, oatmeal agar medium, etc. Its substrate mycelium is relatively long, branched and 0.4—0.6 μm in diameter. No fragmentation of mycelium is observed. The aerial mycelia formed on the Bennet's agar medium or the Hickey-Tresnor's agar medium are white and well developed, non-septated, and 0.6—0.8 μm in diameter. The present strain fails to form spores and sporangium on the substrate mycelium and aerial mycelium so long as the media used are concerned. No formation of sclerotium is observed.

Since the present strain forms relatively long substrate mycelium and aerial mycelium with fine diameters, it is a strain belonging to *Actinomycetes.* Since the strain has the cell wall composition of type III and the whole-cell sugar pattern of type B, it may be classified into any of the genera *Actinomadura, Microbispora, Streptosporangium, Spirillospora* and *Planomonospora*, but the genus of the strain K—26 has not been identified because of the failure of observing spore and sporangium. The present strain has

been deposited as a kind of *Actinomycetes* under FERM P—5889 in the Fermentation Research Institute of Agency of Industrial Science and Technology, and has also been deposited under NRRL 12379 in ARS Culture Collection in the United States of America.

For the production of K—4, microorganisms belonging to actinomycetes, the genus *Actinomadura* are used. An example of suitable strains is a strain K—4 isolated by the present inventors from a soil sample in Kagoshima City in Kagoshima Prefecture, Japan.

Morphological, cultural and physiological characteristics of the strain determined according to the procedure described in detail by Searing et al in Intern. J. System. Bacteriol. *16* 313—340 (1966) will be described below:

I. Morphological characteristics:

The strain hardly grows on a chemically defined medium such as glucose-asparagine agar medium, starch-inorganic salt agar medium, etc., but grows vigorously on a natural nutrient medium such as yeast extract-malt extract agar medium, glucose-yeast extract agar medium, etc. with good formation of white aerial mycelia. Substrate mycelium is relatively short, branched, 0.4—0.6 μm in diameter, and sometimes fragmented at tip ends. Aerial mycelium is well developed, branched and 0.6—0.8 μm in diameter.

Chains of up to 10 spores, which are hooked or spiral, are borne on the sporophores simply branched from the aerial mycelia, and sometimes are tightly closed forming pseudosporangia. The spores are oval in shape, 0.6—0.7 μm × 0.8—1.0 μm in size, spiny surfaced and have no flagella. No formation of sporangiospora, sclerotium, etc. is observed.

II. Growth state on various media

Growth and color characteristics when cultivated on various culture media at 28°C for 2 weeks are given below. Color identification is made in accordance with the color classification of "Color Harmony Manual" (Container Corporation of America). No soluble pigment is observed on all the media used.

1. Glucose-asparagine agar medium
   Growth: very poor

2. Glycerol-asparagine agar medium
   Growth: very poor

3. Starch-inorganic salt agar medium
   Growth: very poor

4. Egg-albumin medium
   Growth: very poor

5. Sucrose-nitrate agar medium
   Growth: poor
   Reverse color: light ivory (2 ca)
   Aerial mycelium: poor, pearl shell tint (3 ba)

6. Glycerol-calcium malate agar medium
   Growth: poor
   Reverse color: colorless
   Aerial mycelium: very poor, white (a)

7. Nutrient agar medium
   Growth: moderate
   Reverse color: amber (3 lc)
   Aerial mycelium: none

8. Yeast extract-malt extract agar medium
   Growth: good
   Reverse color: light amber (3 ic)
   Aerial mycelium: moderate to abundant, white (a)

9. Oatmeal agar medium
   Growth: moderate
   Reverse color: light wheat (2 ea)
   Aerial mycelium: very poor, white (a)

10. Bennet's agar medium
    Growth: moderate
    Reverse color: gold (2 ic)
    Aerial mycelium: moderate, white (a)

11. Emerson's agar medium
    Growth: good
    Reverse color: amber (3 nc)
    Aerial mycelium: poor, white (a)

12. Glucose-yeast extract agar medium
    Growth: good
    Reverse color: amber (3 nc)
    Aerial mycelium: abundant, white (a)

13. Hickey-Tresner's agar medium
    Growth: good
    Reverse color: gold (2 ne)
    Aerial mycelium: abundant, white (a)

14. Peptone-yeast extract-iron agar medium
    Growth: poor
    Reverse color: amber (3 nc)
    Aerial mycelium: none

15. Tyrosine agar medium
    Growth: poor
    Reverse color: light ivory (2 ca)
    Aerial mycelium: very poor, white (a)

III. Physiological properties

Physiological properties of strain K—4 are given below:

In the tests except those on the optimum temperature and actions upon gelatin, milk and cellulose, the strain is cultivated at 28°C for 2 weeks. The temperature is determined after 5 days of cultivation and actions upon milk, gelatin and cellulose are observed after cultivation at 28°C for one month. The present strain grows poorly on the inorganic medium of Pridham-Gottlieb (ISP No. 9). Therefore, the test of utilization of a carbon source is carried out on Ludemann's medium (N.Y. Acad. Sci. *33* 207, 1971), and the result is shown below.

1) Utilization of carbon source: L- and D-arabinose, D-xylose, D-glucose, D-fructose, L-rhamnose, D-galactose, sucrose, mannose, ribose, starch, and D-mannitol are utilized. Raffinose, melizitose, and glycerol are not utilized. Melibiose, inositol, and lactose are slightly utilized.

| | |
|---|---|
| 2) Gelatin liquefaction: | negative |
| 3) Action upon milk: | neither coagulation nor liquefaction |
| 4) Decomposition of cellulose: | weakly positive |
| 5) Hydrolysis of starch: | positive |
| 6) Optimum growth pH: | 6.5—7.8 |
| 7) Optimum growth temperature: | 28°C—37°C |
| 8) Formation of tyrosinase: | negative |
| 9) Formation of melanoid pigment: | negative |

IV. Cell wall composition

As amino acids in the cell wall, *meso*-diaminopimelic acid, alanine, and glutamic acid are contained, and neither LL-diaminopimelic acid nor glycine is contained. On the other hand, by analysis of sugars in the whole cells according to the method of Lechevalier et al (The actiromycetales, Gustav Fisher Verlag, Jena, 311—316, 1970), ribose, glucose, galactose, madurose, etc. are detected, and neither arabinose nor xylose

is detected. According to the classification of Lechevalier et al (Intern. J. System. Bacteriol. *20* 435—443, 1970), the strain K—4 has cell walls of type III and the type B whole cell sugar pattern.

The present strain is classified into the genus *Actinomadura* of actinomycetes, in view of the spore chain formed on the aerial mycelium and the type of cell wall, etc.

Many strains of the genus *Actinomadura* have been recently reported. The present strain is compared with twenty-four strains in approved list of bacterial name (Intern. J. System. Bacteriol. *30* 225—420, 1980) especially with those which produce yellow substrate mycelia, white aerial mycelia and spores with spiny surfaces, and fail to produce soluble pigment. The present strain is obviously different from all the strains except *Actinomadura cremea.* However, *Actinomadura cremea* is reported to have spores with rough surface, but the present strain has a spiny surface. Therefore, these two strains are different from each other. Thus, the strain K—4 is designated as *Actinomadura spiculosospora* nov. sp. K—4, and deposited as FERM P—6101 in the Fermentation Research Institute of Agency of Industrial Science and Technology.

The present strains K—26 and K—4 can be mutated by various mutational treatments such as ultraviolet irradiation, $Co^{60}$ irradiation, X-ray irradiation and treatment with various mutagents as in the case of other actinomycetes, and all the strains having an ability to produce the K—26 or K—4 substance, even though thus mutated, can be used in the present invention.

Cultivation of K—26 or K—4 producing strains

Ordinary procedures for cultivating actinomycetes are used for cultivation of the present K—26 or K—4 producing strains.

Either a synthetic medium or a natural medium may be used so long as it properly contains carbon source(s), nitrogen source(s), inorganic salt(s), etc. As the carbon source, glucose, starch, mannose, fructose, sucrose, molasses, etc. are used alone or in combination. Hydrocarbons, alcohols, organic acids, etc. can also be used, depending upon the assimilability of the microorganisms. As the nitrogen source, inorganic or organic nitrogen-containing compounds such as ammonium chloride, ammonium sulfate, urea, ammonium nitrate and sodium nitrate, and natural nitrogen source such as peptone, meat extract, yeast extract, dry yeast, corn steep liquor, soybean meal, casamino acid and soluble vegetable protein are used alone or in combination. As the inorganic salts, sodium chloride, potassium chloride, calcium carbonate, phosphates, etc. can be added to the medium. Organic or inorganic substances capable of promoting growth of the present strains or production of K—26 or K—4 can be added thereto, if desired.

As the cultivation method, a liquid cultivation, particularly a submerged stirring cultivation, is most suitable. Cultivation is preferably carried out at a temperature of 25—40°C and at a pH of around neutrality. K—26 or K—4 is formed and accumulated in the culture liquor usually after 3—5 days of liquid cultivation. When the accumulation in the culture liquor reaches a maximum, cultivation is discontinued and the desired substance is isolated and purified from the filtrate of the culture liquor obtained by filtering off the cell bodies.

Isolation and purification of K—26 or K—4

Ordinary procedure for isolating metabolic products of microorganisms from a filtrate or culture liquor is used for isolation and purification of K—26 or K—4. That is, such procedures as adsorption and desorption by active carbon, Diaion HP—10 (adsorption resin made by Mitsubishi Kasei Kogyo Co., Ltd., Japan), etc., column chromatography by various ion exchange resins, cellulose column chromatography, silica gel column chromatography, QAE-Sephadex column chromatography, DEAE-Sephadex A—25 column chromatography, Sephadex LH—20 column chromatography (QAE-Sephadex and DEAE-Sephadex A—25 are anion exchange resins, and Sephadex LH—20 is molecular sieve made by Pharmacia Fine Chemicals Inc., Sweden), etc. can be used in a proper combination. One example of purification of K—26 is given below. The filtrate of culture liquor is adjusted to pH 3 with hydrochloric acid, and then passed through a column of Diaion HP—10. The resin is adequately washed with water and eluted with aqueous 50% methanol. Fractions containing K—26 are collected and methanol is distilled off under reduced pressure. Then, the solution is adjusted to pH 7.0 with aqueous 2N sodium hydroxide, and passed through a column of Diaion HPA—10 ($Cl^-$) (anion exchange resin made by Mitsubishi Kasei Kogyo Co., Ltd. Japan). The resin is adequately washed with water and eluted with aqueous 1M sodium chloride. Fractions containing K—26 are collected and adjusted to pH 3 with 2N hydrochloric acid, and passed through a column of Diaion HP—10. The resin is adequately washed with water, and eluted with aqueous 50% methanol. Fractions containing K—26 are collected and concentrated under reduced pressure, and freeze-dried, whereby a crude powder is obtained. The crude powder is adequately admixed with cellulose powder, and the resulting mixture is placed on a column of cellulose which is suspended in aqueous 70% isopropanol in advance. Elution is carried out with aqueous 70% isopropanol. Fractions containing K—26 are collected, concentrated under reduced pressure and then freeze-dried. The freeze-dried preparation is dissolved in 0.15M phosphate buffer (pH 7.5) and placed on a column of DEAE-Sephadex A—25 packed using the same buffer as above. Elution is carried out with the same buffer. Fractions containing K—26 are collected, adjusted to pH 3 with 2N hydrochloric acid and passed through a column of Diaion HP—10. The resin is adequately washed with water and eluted with aqueous 50% methanol. The eluate is concentrated under reduced pressure and freeze-dried, whereby a partially purified powder is obtained. The thus obtained preparation is further purified by Sephadex LH—20 column chromatography (developing solvent:

aqueous 50% methanol). Fractions containing K—26 are collected, concentrated under reduced pressure, and then freeze-dried. The dried preparation is then adequately admixed with silica gel, and the resulting mixture is placed on a column of silica gel packed using the upper layer of n-butanol : n-propanol : water = 2:1:3, and eluted with the same solvent. Fractions containing K—26 are collected, concentrated under reduced pressure to dryness. The residue is dissolved in a small amount of water to remove the insoluble materials, and then freeze-dried, whereby a white powder of K—26 is obtained. In the foregoing purification process, K—26 is detected with Rydon-Smith or iodine reaction.

One example of purification of K—4 is given below.

The filtrate of culture liquor is adjusted to pH 7 with hydrochloric acid, and then passed through a column of Diaion HP—10. The resin is washed with water and eluted with aqueous 50% (V/V) methanol. Fractions containing K—4 are collected, concentrated under reduced pressure and then freeze-dried, whereby a crude powder is obtained. The crude powder is placed on a column of cellulose powder suspended in aqueous 70% (V/V) isopropanol and eluted with the same solvent. Fractions containing K—4 are collected and concentrated under reduced pressure to remove isopropanol, and pH is adjusted to 7.5 with dilute aqueous sodium hydroxide, etc. The resulting solution is charged onto a column of DEAE-Sephadex A—25 equilibrated with 0.01M phosphate buffer (pH 7.5). The column is washed with the same buffer, and then elution is carried out with 0.5M phosphate buffer (pH 7.5). Fractions containing K—4 are collected and passed through a column of Diaion HP—10. The column is washed with water and elution is carried out with aqueous 50% (V/V) methanol. Fractions containing K—4 are allowed to stand in a cold room (4°C), whereby fine crystalline powder of K—4 is deposited. In the foregoing purification process, K—4 is detected with Rydon-Smith or iodine reaction.

Physical and chemical properties of K—26 thus obtained are shown below.

| | |
|---|---|
| State: | amorphous white powder. Its aqueous solution is acidic. |
| Melting point: | no distinct melting point is exhibited up to 300°C, at which point it turns brown. |
| Specific rotation: | $[\alpha]_D^{20} = -4.8°$ (c = 0.1, $H_2O$) |
| Solubility: | readily soluble in alkaline water; soluble in water; sparingly soluble in methanol and ethanol, and insoluble in ethyl acetate, chloroform, hexane. |
| Color reaction: | positive to each of Rydon-Smith and iodine reactions and negative to each of anthrone, aniline, diphenylamine, Ehrlich and nitroprusside reactions. |

Constituent amino acid:

K—26 is hydrolyzed in 6N hydrochloric acid at 105°C for 16 hours and the resulting mixture is analyzed by an amino acid automatic analyzer, whereby isoleucine, tyrosine and an unidentified ninhydrin reaction-positive substance are detected.

Separately, a solution of K—26 in 20% hydrochloric acid is stirred at 110°C for 21 hours and then concentrated under reduced pressure. The residue is chromatographed on a column of silica gel (developing solvent: isopropanol : chloroform : concentrated aqueous ammonia = 3:1:2) to give isoleucine, tyrosine and the aforesaid ninhydrin reaction-positive substance.

The absolute configurations of the isoleucine and tyrosine thus obtained are determined as follows according to the method reported in M. Hasegawa et al, Analytical Biochemistry, *63*, 308 (1975).

(1) The isoleucine thus obtained is reacted with l-menthol in the presence of dried hydrogen chloride to form an l-menthyl ester, which is treated with trifluoroacetic acid to protect the amino group with a trifluoroacetyl group and analyzed by gas chromatography.

The above procedure is repeated using authentic samples of DL-isoleucine and L-isoleucine. As the result, isoleucine from K—26 is identified as L-isoleucine.

(2) The tyrosine thus obtained is converted to an N-trifluoroacetyl-l-menthyl ester thereof in the same manner as described in the above item (1).

The ester is treated with bis-trimethylsilyltrifluoroacetamide to protect the phenolic hydroxyl group with a trimethylsilyl group and analyzed by gas chromatography. The above procedure is repeated using authentic DL-tyrosine and L-tyrosine.

As the result, tyrosine from K—26 is identified as L-tyrosine.

(3) The unidentified ninhydrid reaction-positive substance thus obtained is determined as (−)-1-amino-2-(4-hydroxyphenyl)ethylphosphonic acid (hereinafter referred to as tyrosine-P) based on its $^1$HNMR, $^{13}$CNMR, $^{31}$PNMR, mass spectrum and specific rotation.

Terminal amino acid analysis:

Tyrosine-P is detected by C-terminal analysis according to hydrazine decomposition procedure. No N-terminal amino acid is detected by N-terminal analysis according to DNP formation procedure.

Ultraviolet absorption spectrum: Fig. 1

$\lambda_{max}^{H_2O}$ = 278 nm ($E_{1cm}^{1\%}$ = 46)
281 nm ($E_{1cm}^{1\%}$ = 42, sh)

(1) neutral and (2) acidic

$\lambda_{max}^{H_2O}$ = 294 nm ($E_{1cm}^{1\%}$ = 56.5)
(3) alkaline

Infrared absorption spectrum (KBr): Fig. 2

PMR spectrum ($D_2O$): Fig. 3

CMR spectrum: Fig. 4 (measured at pD 4.0)

Mass spectrum:

Mass spectrum of the hexatrimethylsilyl derivative obtained by treating K—26 with bistrimethylsilyl-trifluoroacetamide and pyridine shows $M^+$ = m/Z 967.

Rf values of K—26 on thin layer chromatography by various developing solvents are shown in Tables 1 and 2, where detection is carried out according to Rydon-Smith reaction.

TABLE 1

Cellulose thin layer chromatography of K–26

| Developing solvent | Rf value |
|---|---|
| 1. aqueous 70% isopropanol | 0.92 |
| 2. n-butanol : acetic acid : water = 3 : 1 : 1 (V/V) | 0.85 |
| 3. Water-saturated n-butanol | 0.57 |

Thin layer Abicel SF (made by Funakoshi Yakuhin Co., Ltd., Japan).

Development: room remperature, ascending method, 5 hours.

TABLE 2

Silica gel thin layer chromatography of K–26

| | Developing solvent | Rf value |
|---|---|---|
| 1. | n-butanol : acetic acid : water = 4 : 1 : 1 (V/V) | 0.52 |
| 2. | n-butanol : n-propanol : water = 2 : 1 : 3 (V/V), upper layer | 0.45 |
| 3. | n-butanol : ethyl acetate : acetic acid : water = 1 : 1 : 1 : 1 (V/V) | 0.72 |
| 4. | ethyl acetate | 0 |
| 5. | ethyl acetate : methanol = 1 : 1 (V/V) | 0.1 – 0.48 |
| 6. | methanol | 0.7 |
| 7. | chloroform : methanol = 1 : 1 (V/V) | 0 |

Thin layer : Kieselgel 60 (made by Merck & Co., U.S.A.).

Development : Room temperature, ascending method, 5 hours for the solvents 1 – 3 and 1.5 hours for the solvents 4 – 7.

Physical and chemical properties of K—4 thus obtained are shown below.

| | |
|---|---|
| State: | white, fine crystalline powder |
| Specific rotation: | $[\alpha]_D^{20} = -83°C$ (c = 0.1, 0.1N NaOH) |
| Melting point: | no distinct melting point is exhibited up to 300°C |
| Solubility in solvent: | soluble in aqueous 0.1N sodium hydrochloride; sparingly soluble in water and methanol; insoluble in chloroform and n-hexane. |
| Color reaction: | positive to Rydon-Smith reaction, iodine reaction, and ninhydrin reaction, and negative to Ehrlich reaction. |

Constituent amino acid:

K—4 is hydrolyzed in 6N hydrochloric acid at 110°C for 20 hours and the resulting mixture is analyzed by an amino acid automatic analyzer, whereby phenylalanine and an unidentified ninhydrin reaction-positive substance are detected.

Separately, a solution of K—4 in 20% hydrochloric acid is stirred at 110°C for 21 hours and then concentrated under reduced pressure. The residue is chromatographed on a column of silica gel (developing solvent: n-butanol : acetic acid : water = 4:1:1) to give phenylalanine, N-methylvaline and the aforesaid ninhydrin reaction-positive substance.

The absolute configuration of the phenylalanine thus obtained is determined as follows according to the method reported in M. Hasegawa et al, Analytical biochemistry, *63*, 308 (1975).

(1) The phenylalanine thus obtained is reacted with l-menthol in the presence of dried hydrogen chloride to form an l-menthyl ester, which is treated with trifluoroacetic acid to protect the amino group with a trifluoroacetyl group and analyzed by gas chromatography.

The above procedure is repeated using an authentic sample of D-phenylalanine and L-phenylalanine. As the result, phenylalanine from K—4 is identified as L-phenylalanine.

(2) N-methylvaline contained in K—4 racemizes in hydrolysis, and the absolute configuration of the N-methylvaline is not determinable. Its absolute configuration is determined to have L-form by total synthesis of K—4.

(3) The unidentified ninhydrin reaction-positive substance thus obtained is determined as (–)-1-amino-2-(4-hydroxyphenyl)ethylphosphonic acid, that is, tyrosine-P based on its $^1$HNMR, $^{13}$CNMR, $^{31}$PNMR, mass spectrum and specific rotation.

Hydrazine decomposition:

Tyrosine-P is detected by C-terminal analysis according to hydrazine decomposition method.

Ultraviolet absorption spectrum: Fig. 5

Infrared absorption spectrum: Fig. 6

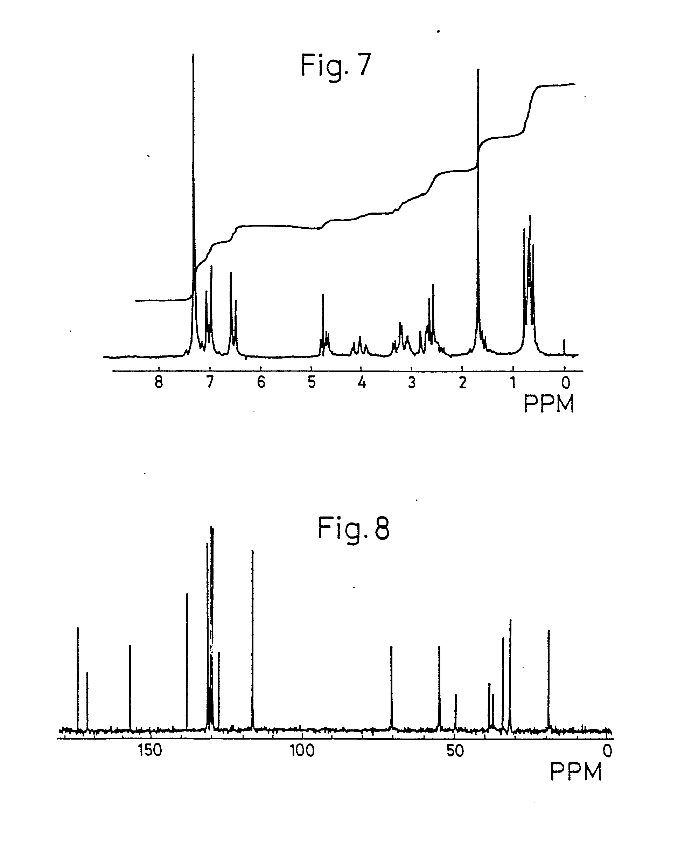

PMR spectrum: Fig. 7

$^{13}$C-NMR spectrum: Fig. 8

Mass spectrum:

Mass spectrum of pentatrimethylsilyl derivative obtained by treating K—4 with bistrimethylsilyltrifluoroacetamide and pyridine shows $M^+ = m/Z$ 837.

Rf values of K—4 on thin layer chromatography by various developing solvents are shown in Table 3.

TABLE 3

K–4 thin layer chromatography

| | Developing solvent | Rf value |
|---|---|---|
| 1. | n-butanol : acetic acid : water (4 : 1 : 1 V/V) | 0.37 |
| 2. | aqueous 70% (V/V) isopropanol | 0.39 |
| 3. | upper layer of n-butanol : n-propanol : water (2 : 1 : 3 V/V) | 0.18 |

Note) Thin layer: Silica gel (Merck Art 5721).

Development: room temperature, ascending method, 4 hours.

Detection: coloring by Rydon-Smith reaction.

Rf values of thin layer chromatography of tyrosine-P contained in hydrolyzate of K—4 by acid are shown in Table 4.

TABLE 4

Thin layer chromatography of tyrosine–P

| | Amino acid | Rf value |
|---|---|---|
| 1. | Phenylalanine | 0.68 |
| 2. | Isoleucine | 0.73 |
| 3. | Tyrosine | 0.42 |
| 4. | Tyrosine–P | 0.33 |

Note) Thin layer: Abicel SF (made by Funakoshi Yakuhin Co., Ltd., Japan).

Developer: n-butanol : acetic acid : water 4 : 1 : 1 (V/V).

Development: room temperature, ascending method, 5 hours.

Detection: ninhydrin reaction.

Hypotensive activity and acute toxicity of Compound [I] are illustrated below as experiments.

Experiment 1

Assessment of hypotensive activity in rats implanted with a catheter:

This experiment is carried out according to the procedure described in "Evaluation of pharmacological effect (1), pharmacological test procedure (II) (Basic Lectures on Development of Drugs V)" compiled by Kyosuke Tsuda et al and published by Chibun Shokan Publishing Co., on October 10, 1971, pages 464—468.

As test animals, three male spontaneously hypertensive rats (SHR) (weight: 300—400 g) are employed as one group. The rats are intraperitoneally anesthetized with 600 mg/kg of urethane and 60 mg/kg of α-chloralose. Trachea is cannulated, and blood pressure is recorded on an ink oscillography through a pressure transducer (Nihon Koden MPU—0.5 made by Nihon Koden Co., Ltd.) from a polyethylene cannula inserted into a left common carotid artery.

Test compound is dissolved in physiological saline solution so that a dosage can be 0.1 ml/100 g, and intravenously administered from a cannula inserted in a left femoral vein, and the blood pressure change is measured.

Changes in mean blood pressure are shown in Table 5. Mean blood pressure of 3 rats just before the administration is 143.5 ± 21.3 mm Hg (average value ± standard error).

TABLE 5

| Sample | Dosage (mg/kg, iv) | Before administration | Blood pressure change after administration (mm Hg) | | | |
|---|---|---|---|---|---|---|
| | | | 10 min. | 30 min. | 60 min. | 120 min. |
| K—26 | 10 | 0 | –14 | –20 | –30 | + 8 |
| ,, | 30 | 0 | –50 | –50 | –53 | –22 |

It is seen from the foregoing results that K—26 has a hypotensive activity.

Experiment 2

Three dd-strain mice weighing 25 ± 1 g are used as one group. K—26 is dissolved in water at a concentration of 15 mg/ml and one ml of the solution is intravenously administered to the mouse (600 mg/kg administration). Three days after the administration, no death is observed in both K—26 administered group and water administered group.

Pharmaceutical compositions of the present invention are described below.

It is obvious from the foregoing experimental data that K—26 has a hypotensive activity.

In view of the hypotensive activity, Compound [I] may be used in various formulation for administration. Pharmaceutical compositions of the present invention are prepared by uniformly mixing an effective amount of Compound [I] with pharmacologically acceptable excipient(s). According to formulation suitable for administration, the excipient may take various forms. It is desirable that the pharmaceutical compositions are administered suitably by injection.

In preparation of the compositions for injection, Compound [I] is dissolved in water. Alternatively, Compound [I] is dissolved in an alkaline solution such as 0.1N aqueous sodium hydroxide, and the solution is adjusted to suitable pH with an acid such as 0.1N hydrochloric acid and diluted with water to a defined volume to prepare an injection. Solubilizing agent(s) may be used in preparation of an injection. Such solubilizing agents include surfactant such as hydrogenated polyoxyethylene castor oil; injectable solvent such as propylene glycol, N,N-dimethylacetamide and ethanol. The active ingredient is administered by injection in a dose of 0.1—100 mg per day for an adult (body weight: 50 kg).

Certain specific embodiments of the invention are illustrated by the following representative examples and reference examples.

Example 1

As a seed strain, *Actinomycetes* K—26 (FERM P—5889) is used, and as a first seed medium, a medium containing 1 g/dl glucose, 1 g/dl soluble starch, 0.3 g/dl beef extract, 0.5 g/dl yeast extract, 0.5 g/dl bactotryptone, and 0.2 g/dl calcium carbonate (pH 7.2 before sterilization) is used. One loopful of the seed strain is inoculated into 14 ml of the seed medium in a 50 ml-large test tube, and cultivated with shaking at 30°C for 11 days. Fourteen milliliters of the seed culture liquor is added to 300 ml of a second seed medium in a 2 l-Erlenmeyer flask having baffles. The composition of the second seed medium is the same as that of the first seed medium. Second seed cultivation is carried out at 30°C for 4 days. Three hundred milliliters of the second seed culture liquor is added to 2.7 l of a third seed medium in a 5 l-jar fermenter. The composition of the third seed medium is the same as that of the first seed medium. Seed cultivation in the 5 l-jar fermenter is carried out at 30°C for 2 days with aeration (3 l/min) and agitation (300 r.p.m.). Two liters of the third seed culture liquor is added to 16 l of a main fermentation medium in a 30 l-stainless steel jar

fermenter. The main fermentation medium contains 4 g/dl soluble starch, 3 g/dl soybean meal, 0.5 g/dl corn steep liquor, 0.05 g/dl dipotassium hydrogen phosphate, 0.05 g/dl magnesium sulfate (heptahydrate), 0.03 g/dl potassium chloride, and 0.3 g/dl calcium carbonate (pH 7.8 before sterilization). The main fermentation is carried out at 30°C for 5 days with aeration (18 l/min) and agitation (350 r.p.m.).

Thirty-five liters of the thus obtained fermentation liquor (volume corresponding to two 30 l-jar fermenters) is adjusted to pH 3.0 with concentrated sulfuric acid, and admixed with about 1.5 kg of Radiolite No. 600 (made by Showa Kagaku Kogyo Co., Ltd., Japan) as a filter aid, and cell bodies are filtered off. Thirty-five liters of the resulting filtrate is passed through a column packed with 2 l of Diaion HP—10. The column is washed with 15 l of water, and eluted with 12 l of aqueous 50% methanol. The first 2 l of the eluate is discarded, while the remaining 10 l is collected, concentrated to 5 l under reduced pressure and adjusted to pH 7.0 with aqueous 2N sodium hydroxide. Then, the solution is passed through a column packed with 500 ml of Diaion HPA—10 ($Cl^-$). The column is washed with 3 l of water and then eluted with 3 l of aqueous 1M sodium chloride. All the eluates are collected and adjusted to pH 3.0 with 2N hydrochloric acid. Then, the solution is passed through a column of 500 ml of Diaion HP—10. The column is washed with 3 l of water and eluted with 3 l of aqueous 50% methanol. The first 500 ml of the eluate is discarded, while the remaining 2.5 l is collected, concentrated to about 1 l under reduced pressure and freeze-dried, whereby about 20 g of crude powder of K—26 is obtained. The crude powder is adequately admixed with 20 g of cellulose powder (Abicel, made by Funakoshi Yakuhin Co., Ltd., Japan), and the resulting mixture is placed on a column of 1 l of cellulose (Abicel: aqueous 70% isopropanol) and eluted with the aqueous 70% isopropanol. The eluate is taken in 18 ml fractions, and K—26 is eluted under fraction Nos. 34—66. The active fractions are collected, concentrated to about 100 ml under reduced pressure and freeze-dried. The freeze-dried preparation is dissolved in 0.15M phosphate buffer (pH 7.5) and placed on a column of 1 l of DEAE-Sephadex A—25 equilibrated with the same buffer. Elution is carried out with the same buffer. The eluate is taken in 18 ml fractions, and K—26 is eluted under fraction Nos. 83—126. The active fractions are collected, adjusted to pH 3.0 with 6N hydrochloric acid, and then passed through a column of 200 ml of Diaion HP—10. The column is washed with 1 l of water and elution is carried out with 1 l of aqueous 50% methanol. All the eluates are collected, concentrated to about 100 ml under reduced pressure and freeze-dried, whereby 450 mg of partially purified powder of K—26 is obtained. Then, the powder is dissolved in 5 ml of aqueous 50% methanol and further purified by Sephadex LH—20 column chromatography (aqueous 50% methanol, column size: 2.3 cm × 90 cm). The eluate is taken in 4 ml fractions, and K—26 is eluted under fraction Nos. 45—68. The active fractions are collected, concentrated to about 20 ml under reduced pressure and then freeze-dried. The dried preparation is adequately admixed with 1.5 g of silica gel (Wako gel C—200 made by Wako Junyaku Co., Ltd.), and the resulting mixture is placed on a column of silica gel suspended in the upper layer of n-butanol : n-propanol : water = 2:1:3 (Wako gel C—200, column size: 2.1 cm × 90 cm), and eluted with the same solvent. The eluate is taken in 3 ml fractions, and K—26 is eluted under fraction Nos. 30—55. The active fractions are collected and concentrated to dryness under reduced pressure. The residue is dissolved in 10 ml of water to remove the insoluble materials and then the solution is freeze-dried, whereby 15 mg of white powder of K—26 is obtained. In the foregoing purification process, detection of K—26 is carried out by means of Rydon-Smith or iodine reaction.

Example 2

As a seed strain, *Actinomadura spiculosospora* nov. sp. K—4 (FERM P—6101) is used and, as a first seed medium, a medium containing 1 g/dl glucose, 1 g/dl soluble starch, 0.3 g/dl beef extract, 0.5 g/dl yeast extract, 0.5 g/dl bactotryptone, and 0.2 g/dl calcium carbonate (pH 7.2 before sterilization). One loopful of the seed strain is inoculated in 10 ml of the seed medium in a 50 ml-large test tube, and cultivated with shaking at 30°C for 5 days. Three milliliters of the seed culture liquor is added to 30 ml of a second seed medium in an 300 ml-Erlenmeyer flask. The composition of the second seed medium is the same as that of the first seed medium. Second seed cultivation is carried out with shaking at 30°C for 2 days. Thirty milliliter of the seed culture liquor is added to 300 ml of a third seed medium in a 2 l-Erlenmeyer flask with baffles. The composition of the third seed medium is the same as that of the first seed medium. The third seed cultivation is carried out with shaking at 30°C for 3 days. Zero point nine liters (corresponding to the volume of three flasks) of the third seed culture liquor is added to 15 l of main fermentation medium in a 30 l-stainless steel jar fermenter. The main fermentation medium contains 4 g/dl soluble starch, 3 g/dl soybean meal, 0.5 g/dl corn steep liquor, 0.05 g/dl dipotassium hydrogen phosphate, 0.05 g/dl magnesium sulfate (heptahydrate), 0.03 g/dl potassium chloride, and 0.3 g/dl calcium carbonate (pH 7.8 before sterilization). The main fermentation is carried out at 30°C for 5 days with aeration (15 l/min) and agitation (300 r.p.m.). The thus obtained culture liquor is admixed with about 1 kg of filter aid, Radiolite No. 600 (made by Showa Kagaku Kogyo Co., Ltd., Japan), and filtered under reduced pressure to obtain 14 l of the filtrate. The filtrate is adjusted to pH 7.0 with hydrochloric acid, and passed through a column of 1 l of Diaion HP—10. The column is washed with water and eluted with aqueous 50% (V/V) methanol. Fractions containing K—4 are concentrated under reduced pressure to remove methanol, and then freeze-dried to obtain 13.0 g of crude brown powder. Thirteen point zero grams of the thus obtained crude K—4 powder is placed on a column of about 1 l of Abicel (cellulose made by Funakoshi Yakuhin Co., Ltd., Japan) which is suspended in an aqueous 70% (V/V) isopropanol and charged into the column uniformly in advance. Then, it is eluted with the same solvent. Fractions containing K—4 are collected and concentrated under reduced

pressure to remove isopropanol. The thus obtained concentrated solution is adjusted to pH 7.5 with sodium hydroxide. The concentrated solution is charged onto a column of 500 ml of DEAE-Sephadex A—25 equilibrated with 0.01M phosphate buffer (pH 7.5) in advance. Then, the column is washed with the same buffer, and elution is carried out with 0.5M phosphate buffer (pH 7.5).

Fractions containing K—4 are collected and adjusted to pH 7.0 with hydrochloric acid, and then passed through a column of 100 ml of Diaion HP—10. The column is washed with water and elution is carried out with aqueous 50% (V/V) methanol. Fractions containing K—4 are allowed to stand in a cold room (4°C), whereby fine crystalline white powder of K—4 is deposited. Thus, 6.5 mg of fine crystalline white powder of K—4 is obtained.

The thus obtained K—4 has the physical and chemical properties as given above.

In the foregoing process, detection of K—4 is carried out by means of Rydon-Smith or iodine reaction.

Example 3

*Step—1:*

Forty-one milligrams of diethyl N-(N-t-butoxycarbonyl-O-benzyl-L-tyrosyl)-(−)-1-amino-2-(4-benzyloxyphenyl) ethylphosphonate is dissolved in 4 ml of ethyl acetate, and admixed with 1 ml of 10%-hydrochloric acid-methanol solution, and the mixture is stirred at room temperature for one day. Then, 50 ml of ethyl acetate is added thereto, and the mixture is washed with an aqueous sodium bicarbonate solution, and then with water, and dried over anhydrous sodium sulfate. The mixture is subjected to silica gel thin layer chromatography for fractionation recovery (chloroform : methanol = 95:5), whereby 26 mg of a colorless glass-like substance is obtained (yield: 73%).

Physical properties of the thus obtained compound are given below:

$[\alpha]_D^{26} = -40.4°$ (c 0.92, MeOH)
IR ($CHCl_3$): 3340, 1679, 1505, 1231, 1044, 1019 $cm^{-1}$

The mass spectrum of the substance shows a molecular ion peak at m/Z 616.

The substance is thereby identified as diethyl N-(O-benzyl-L-tyrosyl)-(−)-1-amino-2-(4-benzyloxyphenyl) ethylphosphonate.

*Step—2:*

Twenty-six milligrams of the compound obtained in Step—1, 7 mg of N-acetyl-L-isoleucine and 5 mg of N-hydroxysuccinimide are dissolved in 1 ml of anhydrous ethylene glycol dimethyl ether, and the solution is stirred on sodium chloride-ice bath. The solution is admixed with 9 mg of DCC and allowed to stand to raise the temperature to room temperature, and subjected to reaction for 26 hours. The formed N,N′-dicyclohexylurea is removed therefrom, and the solvent is distilled off under reduced pressure. The residue is subjected to silica gel thin layer chromatography for fractionation recovery (chloroform : methanol = 97:3), whereby 15 mg of the desired compound is obtained as a white solid (yield: 46%). Physical properties of the thus obtained compound are given below.

Melting point: 145—147°C
$[\alpha]_D^{26} = -46.4°$ (c 0.53, MeOH)
IR ($CHCl_3$): 3290, 1650, 1506, 1230, 1019 $cm^{-1}$
$^1$H-NMR ($CD_3OD$): main peaks are δ: 0.67 (d, 3H), 0.78 (t, 3H), 1.30 (t, 6H), 1.91 (s, 3H), 4.1 (m, 4H), 5.00 (s, 4H), 6.84 (d, 2H), 6.86 (d, 2H), 7.09 (d, 2H), 7.13 (d, 2H), 7.3 (bs, 10H)

Mass spectrum of the substance shows a molecular ion peak at m/Z 771.

The substance is thereby identified as diethyl N-(N-acetyl-L-isoleucyl-O-benzyl-L-tyrosyl)-(−)-1-amino-2-(4-benzyloxyphenyl) ethylphosphonate.

*Step—3:*

Fifteen milligrams of the compound obtained in Step—2 is dissolved in 2 ml of methanol, admixed with 10 mg of 10% palladium-carbon, and subjected to catalytic reduction at room temperature for 3.5 hours. The catalyst is filtered off, and the filtrate is subjected to distillation under reduced pressure. Then, the residue is dissolved in 50 ml of a mixed solvent of chloroform : methanol (9:1) and the solution is passed through a rather short column of silica gel chromatography. Eluate is subjected to distillation under reduced pressure, and the residue is recrystallized from methanol, whereby 10 mg of the desired compound is obtained as colorless needles (yield: 86%).

Physical properties of the thus obtained compound are given below:

Melting point: 292—294°C (dec.)
$[\alpha]_D^{22} = -66.5°$ (c 0.10, MeOH)
IR (KBr): 3240, 1633, 1544, 1199, 1020 $cm^{-1}$
$^1$H-NMR ($CD_3OD$): main peaks are δ: 0.71 (d, 3H), 0.82 (t, 3H), 1.31 (t, 6H), 1.94 (s, 3H), 4.1 (m, 4H), 6.64 (d, 2H), 6.66 (d, 2H), 7.00 (d, 2H), 7.04 (d, 2H)

Mass spectrum of the substance shows a molecular ion peak at m/Z 591.

The substance is thereby identified as diethyl N-(N-acetyl-L-isoleucyl-L-tyrosyl)-(−)-1-amino-2-(4-hydroxyphenyl) ethylphosphonate.

*Step—4:*

Two milligrams of the compound obtained in Step—3 is dissolved in 0.3 ml of a 20% hydrobromic acid-acetic acid solution and the resultant solution is left standing at room temperature for 4 hours. The reaction solution is admixed with 5 ml of water, adjusted to pH 2 with an aqueous 2N caustic soda solution, and passed through a column of HP—20 (nonionic porous resin made by Mitsubishi Kasei Kogyo Co., Ltd., Japan). After the column is washed with water, elution is carried out with an aqueous 0.1N-ammoniacal 50% methanol solution. The eluate is subjected to distillation under reduced pressure, whereby 1 mg of a crude product is obtained. The crude product is dissolved in a small amount of methanol, and the solution is subjected to high speed liquid chromatography [μ Bondapack $C_{18}$ (made by Waters Co.,), 0.01M ammonium acetate (pH 5.0) : acetonitrile = 9:1, 4 ml/min]. Portions whose retention time is 2.7 minutes are collected, concentrated and freeze-dried, whereby the purified desired compound is obtained as a white solid.

Physical properties of the thus obtained compound are shown below:

Melting point: 300°C or higher

$^1$H-NMR ($D_2O$, pD 4.5): main peaks are δ: 0.65 (d, 3H), 0.81 (t, 3H), 2.00 (s, 3H), 3.99 (d, 1H), 4.30 (bt, 1H), 4.65 (dd, 1H), 6.83 (d, 4H), 7.14 (d, 2H), 7.20 (d, 2H)

The substance is thereby identified as N-(N-acetyl-L-isoleucyl-L-tyrosyl)-(−)-1-amino-2-(4-hydroxyphenyl) ethylphosphonic acid.

Example 4

*Step—1:*

Seventy-five milligrams of diethyl N-(N-benzyloxycarbonyl-L-phenylalanyl)-(−)-1-amino-2-(4-benzyloxyphenyl)ethylphosphonate is dissolved in 5 ml of methanol-acetic acid (4:1) and admixed with 40 mg of 10% palladium-carbon. The mixture is subjected to catalytic reduction for 8 hours. After the catalyst is filtered off, the filtrate is subjected to distillation under reduced pressure, and the residue is subjected to silica gel thin layer chromatography for fractionation recovery (chloroform : methanol = 9:1), whereby 40 mg of the desired compound is obtained as a colorless glass-like substance (yield: 83%).

Physical properties of the thus obtained compound are given below:

$[\alpha]_D^{26} = -63.0°$ (c 1.36, MeOH)

IR ($CHCl_3$): 3320, 1660, 1508, 1226, 1041, 1020 $cm^{-1}$

$^1$H-NMR ($CD_3OD$): main peaks are δ: 1.29 (m, 6H, 2.5—3.7 (m, 5H), 4.1 (m, 4H), 4.56 (m, 1H), 6.70 (d, 2H), 7.05 (d, 2H), 7.2 (5H)

Mass spectrum of the substance shows a molecular ion peak at m/Z 420.

The substance is thereby identified as diethyl N-(L-phenylalanyl)-(−)-1-amino-2-(4-hydroxyphenyl) ethylphosphonate.

*Step—2:*

Forty milligrams of the compound obtained in Step—1, 26 mg of N-benzyloxycarbonyl-N-methyl-L-valine, and 11 mg of N-hydroxysuccinimide are dissolved in 1 ml of anhydrous ethylene glycol dimethyl ether, and the solution is stirred on sodium chloride-ice bath. The solution is admixed with 22 mg of DCC and stirred for 3 hours. Then the mixture is heated to room temperature and stirred for 26 hours. The formed N,N′-dicyclohexylurea is filtered off, and the filtrate is admixed with 50 ml of ethyl acetate. The organic layer is washed with an aqueous sodium bicarbonate solution, and then with water, and dried over anhydrous sodium sulfate. The solvent is distilled off under reduced pressure, and the residue is subjected to silica gel thin layer chromatography for fractionation recovery (chloroform : methanol = 96:4), whereby 17 mg of the desired compound is obtained as a colorless glass-like substance (yield: 27%).

Physical properties of the thus obtained compound are shown below:

$[\alpha]_D^{26} = -95.7°$ (c 0.68, MeOH)

IR ($CHCl_3$): 3410, 1674, 1509, 1222, 1040, 1020 $cm^{-1}$

$^1$H-NMR ($CD_3OD$): main peaks are δ: 0.76 (d, 6H), 1.33 (t, 6H), 2.52 (bs, 3H), 4.12 (m, 4H), 5.14 (s, 2H), 6.66 (d, 2H), 7.05 (d, 2H), 7.10 (s, 5H), 7.37 (s, 5H)

Mass spectrum of the substance shows a molecular ion peak at m/Z 667.

The substance is thereby identified as diethyl N-(N-benzyloxycarbonyl-N-methyl-L-valyl-L-phenylalanyl)-(−)-1-amino-2-(4-hydroxyphenyl) ethylphosphonate.

*Step—3:*

Seventeen milligrams of the compound obtained in Step—2 is dissolved in 1 ml of methanol-acetic acid (9:1) and admixed with 10 mg of 10% palladium-carbon. The mixture is subjected to catalytic reduction for one day. The catalyst is filtered off, and the filtrate is subjected to distillation under reduced pressure. The residue is subjected to silica gel thin layer chromatography for fractionation recovery (chloroform : methanol = 9:1), whereby 6 mg of the desired compound is obtained as a colorless glass-like substance (yield: 41%).

Physical properties of the thus obtained compound are shown below:

$[\alpha]_D^{26} = -40.9°$ (c 0.56, MeOH)

IR ($CHCl_3$): 3310, 1673, 1661, 1509, 1227, 1041, 1020 $cm^{-1}$

$^1$H-NMR ($CD_3OD$): main peaks are δ: 0.70 (d, 3H), 0.76 (d, 3H), 1.33 (t, 6H), 1.93 (s, 3H), 2.62 (d, 1H), 4.13 (m, 4H), 6.64 (d, 2H), 7.04 (d, 2H), 7.22 (s, 5H)

Mass spectrum of the substance shows a molecular ion peak at m/Z 533.

The substance is thereby identified as diethyl N-(N-methyl-L-valyl-L-phenylalanyl)-(−)-1-amino-2-(4-hydroxyphenyl) ethylphosphonate.

*Step—4:*

One milligram of the compound obtained in Step—3 is dissolved in 0.3 ml of 20% hydrobromic acid-acetic acid solution, and the solution is left standing at room temperature for 4 hours. By the same procedure as in Example 7, 0.5 mg of a crude product is obtained. The crude product is dissolved in a small amount of an aqueous 0.04N caustic soda solution, and the resultant solution is subjected to high speed liquid chromatography (under the same conditions as in Step—4 in Example 3). Portions whose retention time is 1.8 minutes are collected, concentrated and freeze-dried, whereby the purified desired compound is obtained as a white solid.

Physical properties of the thus obtained compound are shown below:

Melting point: 300°C or higher

$^1$H-NMR ($D_2O$, pD 11.9): main peaks are δ: 0.71 (d, 3H), 0.79 (d, 3H), 1.74 (s, 3H), 2.69 (d, 1H), 4.08 (bt, 1H), 6.53 (d, 2H), 7.01 (d, 2H), 7.34 (s, 5H)

The substance is thereby identified as N-(N-methyl-L-valyl-L-phenylalanyl)-(−)-1-amino-2-(4-hydroxyphenyl) ethyphosphonic acid.

## Example 5

One hundred milligrams of K—26 is dissolved in water and the solution is diluted to a volume of 100 ml. The solution is filtered under sterile condition with a membrane filter. The filtrate is poured in an ampule by portions of 2.5 ml. The ampule is sealed and sterilized at 121°C for 20 minutes with high pressure steam to prepare an injection.

## Example 6

One hundred milligrams of K—26 is dissolved in 0.1N aqueous sodium hydroxide, and the solution is adjusted to pH 9 with 0.1N hydrochloric acid and diluted to a volume of 20 ml with water. Thereafter, the same procedure as described in Example 5 is repeated to prepare an injection containing 2.5 mg of K—26 per ampule (0.5 ml).

## Example 7

Five hundred milligrams of K—4 is dissolved in 0.1N aqueous sodium hydroxide, and the solution is adjusted to pH 9 with 0.1N hydrochloric acid and diluted to a volume of 20 ml with water. The solution is filtered under sterile condition with a membrane filter. The filtrate is poured in an ampule by portions of 5 ml. The ampule is sealed and sterilized at 121°C for 20 minutes with high pressure steam to prepare an injection.

## Example 8

One hundred milligrams of K—4 and 500 mg of surfactant, HCO—60 (made by Nikko Chemicals Co., Ltd., Japan) are dissolved in water. The solution is diluted to a volume of 20 ml with water. The resulting solution is treated in the same manner as in Example 7 to prepare an injection containing 50 mg of K—4 per ampule (10 ml).

## Reference Example 1

*Step—A:*

One point five two grams of 4-hydroxyphenylacetic acid and 1.2 g of caustic soda are dissolved in 30 ml of water-ethanol (2:1), admixed with 2.97 ml of benzyl bromide, and stirred at room temperature for one day. The reaction solution is adjusted to pH 2 with hydrochloric acid, and extracted with ethyl acetate. The

extract is washed with water and dried over anhydrous sodium sulfate. The solvent is distilled off under reduced pressure, and the residue is recrystallized from benzene-n-hexane, whereby 1.71 g of colorless crystals as the desired compound is obtained (yield: 71%). The physical properties of the thus obtained crystals are shown below:

Melting point: 123—124°C
$^1$H-NMR spectrum ($CDCl_3$) δ: 3.56 (s, 2H), 5.02 (s, 2H), 6.87 (d, 2H), 7.17 (d, 2H), 7.33 (s, 5H), 10.7 (bs, 1H)
IR (KBr): 3040, 1685, 1507, 1240 $cm^{-1}$

The substance is thereby identified as 4-benzyloxyphenylacetic acid.

*Steps—B, C and D:*

Six point zero five grams of 4-benzyloxyphenylacetic acid is dissolved in 125 ml of thionyl chloride, and boiled for 4 hours. Excess thionyl chloride is distilled off under reduced pressure, and the residue is dried, whereby 4-benzyloxyphenylacetic acid chloride is obtained. The thus obtained product is then dissolved in 25 ml of anhydrous benzene, and the resulting solution is added dropwise to 8.3 g of triethyl phosphite in a nitrogen gas stream with stirring under ice cooling. Thirty minutes thereafter, temperature is elevated to room temperature, and the solution is stirred for one day, whereby diethyl 4-benzyloxyphenylacetylphosphate is obtained. The substance is unstable and thus is admixed with 50 ml of ethanol and 3 ml of anhydrous pyridine without purification with stirring. Then, the mixture is admixed with 1.92 g of hydroxylamine hydrochloride and stirred at room temperature for 4 hours. The reaction solution is admixed with 200 ml of water and 15 ml of 2N HCl, and extracted with chloroform. The organic layer is washed with water and then dried. The solvent is distilled off under reduced pressure, and the residue is subjected to silica gel column chromatography (chloroform : acetone = 9:1), whereby 5.4 g of the desired product is obtained as a light yellow oily product (yield: 57%).

The physical properties of the thus obtained compound are shown below:
Mass spectrum of the substance shows a molecular ion peak at m/Z 377.

$^1$H-NMR spectrum ($CDCl_3$): main peaks are δ: 1.19 (t, 6H), 3.7—4.2 (m, 6H), 5.02 (s, 2H), 6.86 (d, 2H), 7.26 (d, 2H), 7.36 (bs, 5H).

The substance is thereby identified as diethyl 4-benzyloxyphenylacetylphosphonate oxime.

Eight hundred sixty milligrams of diethyl 4-benzyloxyphenylacetylphosphonate oxime is dissolved in 4.6 ml of formic acid, admixed with 600 mg of zinc dust with stirring under ice cooling, and stirred for 3 hours. The reaction solution is filtered, and the filtrate is admixed with 50 ml of ethyl acetate. The organic layer is washed with a saturated aqueous sodium bicarbonate solution and then with water, and dried with anhydrous sodium sulfate. The solvent is distilled off under reduced pressure, and the residue is subjected to silica gel column chromatography (chloroform : methanol = 98:2), whereby 540 mg of the desired compound is obtained as a colorless solid (yield: 65%).

The physical properties of the thus obtained solid are shown below:

Melting point: 53—55°C
Elemental analysis (%)

| | C | H | N |
|---|---|---|---|
| Found | 62.6 | 6.7 | 3.6% |
| Calculated (as $C_{19}H_{26}NO_4P$) | 62.8 | 7.2 | 3.9% |

Mass spectrum of the substance shows a molecular ion peak at m/Z 363.

$^1$H-NMR ($CDCl_3$): main peaks are δ: 1.33 (t, 6H), 2.98 (m, 1H), 3.17 (m, 2H), 4.16 (m, 4H), 5.03 (s, 2H), 6.91 (d, 2H), 7.15 (d, 2H), 7.35 (bs, 5H)
IR ($CHCl_3$): 1505, 1230, 1046, 1020 $cm^{-1}$

The substance is thereby identified as diethyl 1-amino-2-(4-benzyloxyphenyl) ethylphosphonate.

*Step—E:*

One hundred thirty-eight milligrams of diethyl 1-amino-2-(4-benzyloxyphenyl) ethylphosphonate, 142 mg of N-t-butoxycarbonyl-O-benzyl-L-tyrosine, and 44 mg of N-hydroxysuccinimide are dissolved in 4 ml of anhydrous ethyleneglycol dimethyl ether, and stirred on sodium chloride-ice bath. The solution is admixed with 86 mg of DCC and stirred under cooling for 2 hours, and further at room temperature for 26 hours. The formed N,N-dicyclohexylurea is filtered off, and the filtrate is admixed with 100 ml of ethyl acetate, washed with water, and then dried over anhydrous sodium sulfate. The solvent is distilled off under reduced pressure, and then the residue is subjected to silica gel thin layer chromatography for fractionation recovery, developed with a mixed solvent of chloroform : acetone = 9:1, and separated into

the portion of Rf = 0.39 and the portion of Rf = 0.27. The portion of Rf = 0.39 is collected, and eluted with the same solvent. The solvent is distilled off under reduced pressure, whereby 100 mg of the desired compound is obtained as a colorless, glass-like substance (yield: 73%).

The physical properties of the thus obtained compound are shown below:

$[\alpha]_D^{26} = -40.2°$ (c 0.92, MeOH)
IR ($CHCl_3$): 1673, 1504, 1234, 1042, 1018 $cm^{-1}$
$^1$H-NMR ($CDCl_3$): main peaks are δ: 1.23 (t, 3H), 1.28 (t, 3H), 1.38 (s, 9H), 2.5—4.8 (m, 10H), 4.99 (s, 2H), 5.01 (s, 2H), 6.8—7.1 (m, 8H), 7.36 (bs, 10H)

Mass spectrum of the substance shows a molecular ion peak at m/Z 716.

The substance is thereby identified as diethyl N-(N-t-butoxycarbonyl-O-benzyl-L-tyrosyl)-(−)-1-amino-2-(2-benzyloxyphenyl) ethylphosphonate.

Reference Example 2

*Step—E:*

Four hundred eight milligrams of diethyl 1-amino-2-(4-benzyloxyphenyl) ethylphosphonate, 336 mg of N-benzyloxycarbonyl-L-phenylalanine, and 129 mg of N-hydroxysuccinimide are dissolved in 11 ml of anhydrous ethylene glycol dimethyl ether, and the solution is stirred on sodium chloride-ice bath. The solution is admixed with 255 mg of DCC and stirred for 2.5 hours. Then, the solution is left standing in a refrigerator (about 5°C) for 2.5 days. The formed N,N′-dicyclohexylurea is filtered off, and the filtrate is admixed with 150 ml of ethyl acetate. After the similar treatment to that of Step E of Reference Example 1, the residue is subjected to silica gel column chromatography (chloroform : acetone = 9:1; separation into the portion of Rf = 0.33 and the portion of Rf = 0.25 by silica gel thin layer chromatography with the same solvent), whereby 265 mg of the desired compound of Rf 0.33 is obtained as a colorless, glass-like substance (yield: 73%).

The physical properties of the thus obtained compound are shown below:

$[\alpha]_D^{26} = -48.5°$ (c 1.14, MeOH)
IR ($CHCl_3$): 1715, 1677, 1507, 1231, 1043, 1020 $cm^{-1}$
$^1$H-NMR ($CDCl_3$): main peaks are δ: 1.20 (t, 3H), 1.27 (t, 3H), 2.6—5.0 (m, 10H), 4.95 (s, 2H), 5.01 (s, 2H), 6.82 (d, 2H), 7.08 (d, 2H), 7.1—7.3 (15H)

Mass spectrum of the substance shows a molecular ion peak at m/Z 644.

The present substance is thereby identified as diethyl N-(N-benzyloxycarbonyl-L-phenylalanyl)-(−)-1-amino-2-(4-benzyloxyphenyl) ethylphosphonate.

Reference Example 3

Preparation of (−)-Compound [XII]:

Ninety-three milligrams of diethyl N-(N-t-butoxycarbonyl-O-benzyl-L-tyrosyl)-(−)-1-amino-2-(4-benzyloxyphenyl)ethylphosphonate is added to 26 ml of 6N hydrochloric acid, and heated at 110°C in a sealed tube for 20 hours. After opening the sealing, water is added thereto, and the mixture is evaporated to dryness under reduced pressure. This operation is repeated three times, and then the residue is subjected to silica gel column chromatography (i-propanol : chloroform : concentrated aqua ammonia = 3:1:2), whereby the desired compound is obtained. The compound is dissolved in a small amount of an aqueous 0.2N caustic soda solution, and the solution is passed through a column of Amberlite CG—50 ($H^+$ type) (product of Rohm & Haas Co., U.S.A.), followed by elution with water. After freeze-drying, 22 mg of the desired compound is obtained as a light brown solid (yield: 80%).

The physical properties of the thus obtained solid are given below:

Melting point: 300°C or higher
$[\alpha]_D^{26} = -40 \pm 2°$ (c 0.51, 1.0N-NaOH)
IR (KBr): 3420, 3240, 1610, 1511, 1248, 1146, 1022 $cm^{-1}$
$^1$H-NMR ($D_2O$, pD 7.6) δ: 2.8 (m, 1H), 3.39 (m, 2H), 6.97 (d, 2H), 7.32 (d, 2H)
13C-NMR ($D_2O$, pD 7.6) δ: 35.0, 53.5 (d), 116.7, 129.7 (d), 131.3, 155.5

The substance is thereby identified as (−)-1-amino-2-(4-hydroxyphenyl) ethylphosphonic acid.

Reference Example 4

Preparation of (−)-Compound [XII]:

Seventy-eight milligrams of diethyl N-(N-benzyloxycarbonyl-L-phenyl)-(−)-1-amino-2-(4-benzyloxyphenyl) ethylphosphonate is added to 24 ml of 6N hydrochloric acid. After the similar treatment to that of Reference Example 3, 16 mg of the desired compound is obtained (yield: 61%). The substance is identical with the compound obtained in Reference Example 3 in thin layer chromatography, $^1$H-NMR, IR, specific rotation, etc., and thus is identified as (−)-1-amino-2-(4-hydroxyphenyl) ethylphosphonic acid.

Reference Example 5

Preparation of Compound [II] from (−)-Compound [XII]:

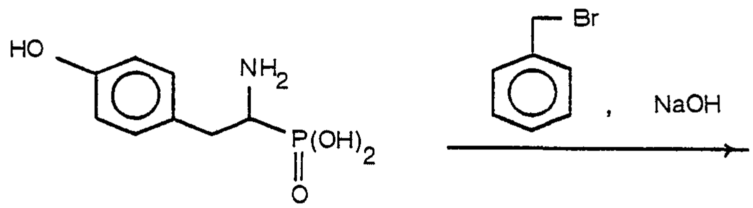


(−)-1-amino-2-(4-hydroxyphenyl) ethylphosphonic acid

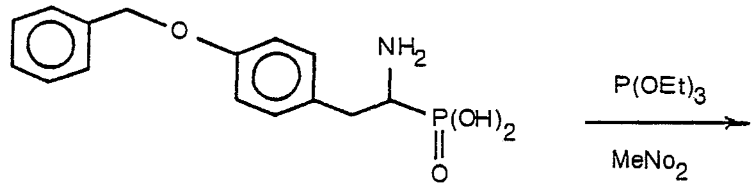


(−)-1-amino-2-(4-benzyloxyphenyl) ethylphosphonic acid

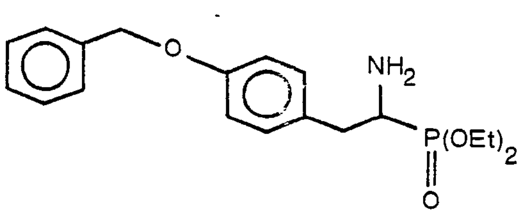

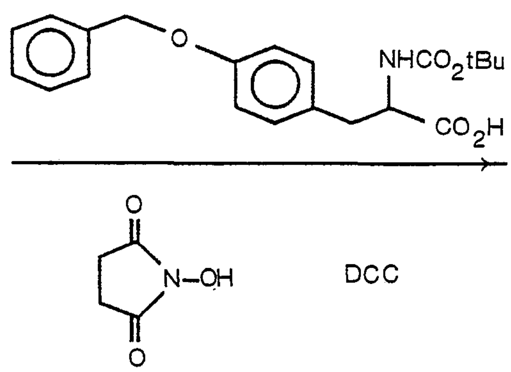


diethyl (− )-1-amino-2-(4-benzyloxy-phenyl) ethylphosphonate

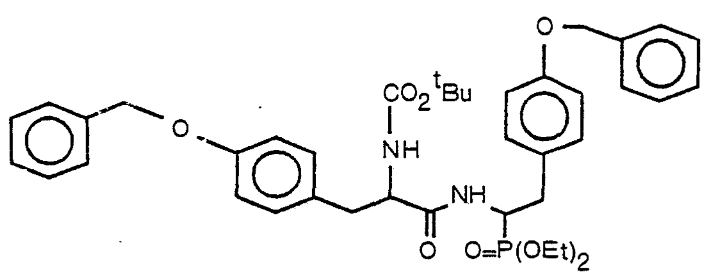

diethyl N−(N-t-butoxycarbonyl-O-benzyl-L-tyrosyl)-(−)-1-amino-2-(4-benzyloxyphenyl) ethylphosphonate

Eighty-three milligrams of (−)-1-amino-2-(4-hydroxyphenyl) ethylphosphonic acid is dissolved in 1.5 ml of an aqueous 1N sodium hydroxide solution, and admixed with 3 ml of ethanol. Then, the solution is admixed with 55 µl of benzyl bromide, and stirred at room temperature for one day. The reaction solution is adjusted to pH 2 with hydrochloric acid, and extracted with ethyl acetate. The extract is washed with water, and dried over anhydrous sodium sulfate. Then, the solvent is distilled off under reduced pressure, whereby the desired compound is obtained (80 mg).

Eighty milligrams of the thus obtained crude (−)-1-amino-2-(4-benzyloxyphenyl) ethylphosphonic acid is dissolved in 5 ml of nitromethane, admixed with 200 µl of triethyl phosphite, and the mixture is heated at 100°C for 12 hours with stirring. The reaction solution is admixed with ethyl acetate, and the organic layer is washed with an aqueous saturated sodium bicarbonate solution, and then with water, and dried over anhydrous sodium sulfate. The solvent is distilled off under reduced pressure, and the residue is subjected to silica gel column chromatography (chloroform : methanol = 98:2), whereby 74 mg of the desired compound is obtained as a colorless solid (yield: 78%).

Seventy-four milligrams of the thus obtained diethyl (−)-1-amino-2-(4-benzyloxyphenyl) ethylphosphonate, 76 mg of N-t-butoxycarbonyl-O-benzyl-L-tyrosine, and 24 mg of N-hydroxysuccinimide are dissolved in 3 ml of anhydrous ethylene glycol dimethyl ether, and the solution is stirred on sodium chloride-ice bath. Then, the solution is admixed with 46 mg of DCC, and stirred under cooling for 2 hours, and further at room temperature for 26 hours. The formed N,N′-dicyclohexylurea is filtered off, and the

filtrate is admixed with 50 ml of ethyl acetate, washed with water, and then dried over anhydrous sodium sulfate. The solvent is distilled off under reduced pressure, and the residue is purified by silica gel thin layer chromatography for fractionation recovery (developing solvent: chloroform : acetone = 9:1), whereby 101 mg of the desired compound is obtained as a colorless glass-like substance (yield: 69%).

The properties of the thus obtained compound is identical with those of diethyl N-(N-t-butoxycarbonyl-O-benzyl-L-tyrosyl)-(−)-1-amino-2-(4-benzyloxyphenyl) ethylphosphonate obtained in Step—E of Reference Example 1.

**Claims**

1. Phosphorus-containing oligopeptides represented by the formula [I]:

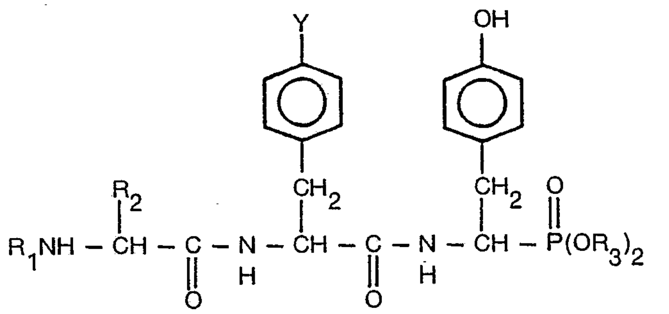

[I]

wherein $R_1$ represents hydrogen, alkyl having 1—6 carbon atoms, unsubstituted or substituted phenylalkyl (wherein the alkyl part has 1—6 carbon atoms, and the substituent is selected from halo, nitro and alkoxy having 1—3 carbon atoms), or $R_4CO$— (wherein $R_4$ represents hydrogen, alkyl having 1—6 carbon atoms, or unsubstituted or substituted phenylalkyl (wherein the alkyl part has 1—6 carbon atoms, and the substituent is selected from halo, nitro and alkoxy having 1—3 carbon atoms), $R_2$ represents alkyl having 1—6 carbon atoms; $R_3$ represents hydrogen, alkyl having 1—6 carbon atoms, or unsubstituted or substituted phenylalkyl (wherein the alkyl part has 1—6 carbon atoms, and the substituent is selected from halo, nitro and alkoxy having 1—3 carbon atoms) and Y represents hydrogen or hydroxyl".

2. Phosphorus containing oligopeptides according to claim 1 wherein the configuration of the radicals derived from the two amino acid are in the L-form and the radical derived from the 1-amino-2-(4-hydroxyphenyl)ethylphosphonic acid has a negative specific rotation.

3. Phosphorus-containing oligopeptides according to claim 2 wherein $R_1$ is $R_4CO$—, $R_2$ is sec-butyl and Y is hydroxyl.

4. Phosphorus-containing oligopeptides according to claim 3 wherein $R_4$ is methyl.

5. Phosphorus-containing oligopeptides according to claim 2 wherein $R_1$ is hydrogen, alkyl or substituted or unsubstituted phenylalkyl, $R_2$ is i-propyl and Y is hydrogen.

6. Phosphorus-containing oligopeptides according to claim 5 wherein $R_1$ is methyl.

7. A process for preparation of Compound [I] as claimed in claim 1 which comprises eliminating the protective group(s) of a compound represented by the formula (V):

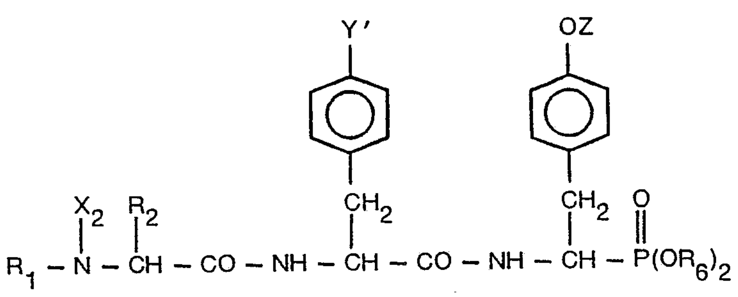

[V]

wherein $X_2$ is hydrogen when $R_1$ is $R_4CO$—, or an amino-protecting group usually used in the peptide synthesis when $R_1$ is hydrogen, alkyl, or substituted or unsubstituted phenylalkyl; Y' represents hydrogen, hydroxyl or OZ; Z represents a hydroxyl-protecting group usually used in the peptide synthesis; $R_6$ has the same meaning as $R_3$ excluding hydrogen; and $R_1$, $R_2$, $R_3$ and $R_4$ have the same meaning as defined in claim 1, and if necessary hydrolyzing the resulting compound.

8. A process for preparation of K—26, namely N-(N-acetyl-L-isoleucyl-L-tyrosyl)-(−)-1-amino-2-(4-hydroxyphenyl) ethylphosphonic acid which comprises cultivating a microorganism belonging to *Actinomycetes* having an ability to produce K—26 in a culture medium, forming and accumulating K—26 in the culture liquor, and recovering K—26 therefrom.

9. A process according to claim 8 wherein the microorganism is *Actinomycetes* K—26 (FERM P—5889, NRRL 12379).

10. A process for preparation of K—4, namely N-(N-methyl-L-valyl-L-phenylalanyl)-(−)-1-amino-2-(4-hydroxyphenyl) ethylphosphonic acid which comprises cultivating a microorganism belonging to the genus *Actinomadura* and having an ability to produce K—4 in a culture medium, forming and accumulating K—4 in the culture liquor, and recovering K—4 therefrom.

11. A process according to claim 10 wherein the microorganism belongs to the species *Actinomadura spiculosospora*.

12. A process according to claim 11 wherein the microorganism is *Actinomadura spiculosospora* nov. sp. K—4 (FERM P—6101).

13. A pharmaceutical composition which comprises an effective amount of Compound [I] as claimed in claim 1 and pharmacologically acceptable excipient(s).

14. A pharmaceutical composition according to claim 13 wherein configurations of the radical derived from the two amino acids are in the L-form and the radical derived from 1-amino-2-(4-hydroxyphenyl) ethylphosphonic acid has a negative specific rotation.

15. A microorganism *Actinomycetes* K—26 (FERM P—5889, NRRL 12379).

16. A microorganism *Actinomadura spiculosospora* nov. sp. K—4 (FERM P—6101).

**Patentansprüche**

1. Phosphorhaltige Oligopeptide der Formel:

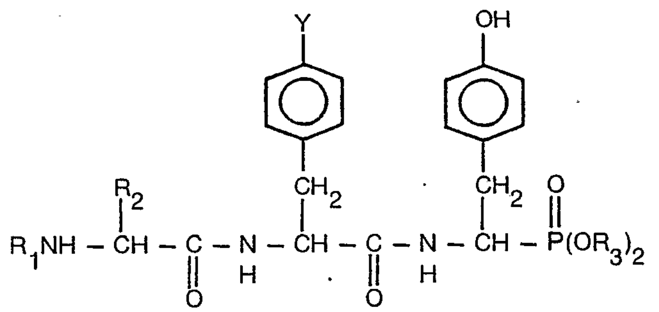

[I]

in der $R_1$ Wasserstoff, ein Alkyl mit 1 bis 6 Kohlenstoffatomen, ein nicht substituiertes oder substituiertes Phenylalkyl (worin der Alkyl-Teil 1 bis 6 Kohlenstoffatome hat und der Substituent aus einer Halo-, Nitro- oder Alkoxygruppe mit 1 bis 3 Kohlenstoffatomen gewählt wird), oder $R_4CO$— (worin $R_4$ Wasserstoff, ein Alkyl mit 1 bis 6 Kohlenstoffatomen oder ein nichtsubstituiertes oder substituiertes Phenylalkyl (worin der Alkyl-Teil 1 bis 6 Kohlenstoffatome hat und der Substituent aus einer Halo-, Nitro- oder Alkoxygruppe mit 1 bis 3 Kohlenstoffatomen gewählt wird) darstellt, $R_2$ ein Alkyl mit 1 bis 6 Kohlenstoffatomen darstellt; $R_3$ Wasserstoff, ein Alkyl mit 1 bis 6 Kohlenstoffatomen oder ein nicht substituiertes oder substituiertes Phenylalkyl (worin der Alkyl-Teil 1 bis 6 Kohlenstoffatome hat und der Substituent aus einer Halo-, Nitro- oder Alkoxygruppe mit 1 bis 3 Kohlenstoffatomen gewählt wird) darstellt und Y Wasserstoff oder Hydroxyl bedeutet.

2. Phosphorhaltige Oligopeptide gemäß Anspruch 1, in dem die Konfiguration der von den zwei Aminosäuren abgeleiteteten Radikale L-Form hat und der von der 1-Amino-2-(4-hydroxyphenyl) ethylphosphonsäure abgeleitete Radikal die negative spezifische Drehung hat.

3. Phosphorhaltige Oligopeptide gemäß Anspruch 2, in denen $R_1$ $R_4CO$—, $R_2$ sek. Butyl und Y Hydroxyl bedeuten.

4. Phosphorhaltige Oligopeptide gemäß Anspruch 3, in denen $R_4$ Methyl bedeutet.

5. Phosphorhaltige Oligopeptide gemäß Anspruch 2, in denen $R_1$ Wasserstoff, Alkyl oder substituiertes oder nicht substituiertes Phenylalkyl, $R_2$ i-Propyl und Y Wasserstoff bedeuten.

6. Phosphorhaltige Oligopeptide gemäß Anspruch 5, in denen $R_1$ Methyl bedeutet.

7. Verfahren zu Herstellung der Verbindung (I) bestehend aus der Eliminierung der Schutzgruppe(n) einer Verbindung gemäß der Formel:

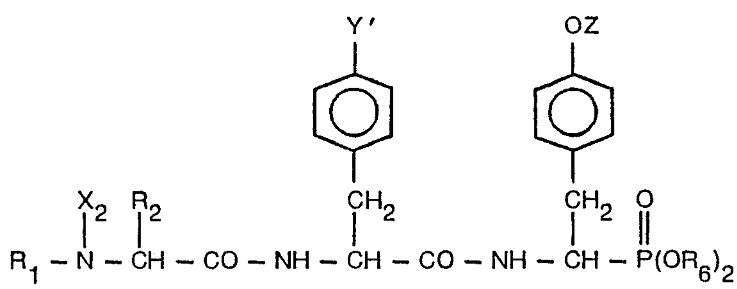

[V]

in der $X_2$ Wasserstoff bedeutet wenn $R_1$ ein $R_4CO$—, oder eine üblicherweise bei der Peptidsynthese eingesetzte Aminoschutzgruppe bedeutet, wenn $R_1$ Wasserstoff, Alkyl, oder substituiertes oder nicht substituiertes Phenylalkyl ist; Y' Wasserstoff, Hydroxyl oder OZ ist, wobei Z eine üblicherweise bei der Peptidsynthese eingesetzte Hydroxyl-Schutzgruppe bedeutet; $R_6$ die gleiche Bedeutung hat wie $R_3$ mit Ausnahme von Wasserstoff; und $R_1$, $R_2$, $R_3$ und $R_4$ die gleiche Bedeutung wie oben haben und wobei ggf. die sich ergebende Verbindung hydrolysiert wird.

8. Verfahren zu Herstellung von K—26, d.i. N-(N-acetyl-L-isoleucyl-L-tyrosyl)-(−)-1-amino-2-(4-hydroxyphenyl)ethylphosphonsäure, das umfaßt: Züchten eines Mikroorganismus, der zu den *Actinomyceten* (Strahlenpilzen) gehört, und die Fähigkeit zur Produktion des K—26 hat, in einem Kulturmedium und der K—26 in der Kulturflüssigkeit bildet und anreichert, und Gewinnung des K—26 daraus.

9. Verfahren gemäß Anspruch 8, in dem es sich bei dem Mikroorganismus um *Actinomycetes* K—26 (FERM P—5889, NRRL 12379) handelt.

10. Verfahren zur Herstellung von K—4, d.i. N-(N-methyl-L-valyl-L-phenylalanyl)-(−)-1-amino-2-(4-hydroxyphenyl)ethylphosphonsäure, das umfaßt: Züchten eines Mikroorganismus aus der Gattung *Actinomadura*, der die Fähigkeit K—4 zu produzieren hat, in einem Kulturmedium, und der K—4 in der Kulturflüssigkeit bildet und anreichert, und Gewinning des K—4 daraus.

11. Verfahren gemäß Anspruch 10, in dem der Mikroorganismus zur Spezies *Actinomadura spiculosospora* gehört.

12. Verfahren gemäß Anspruch 11, in dem es sich bei dem Mikroorganismus um *Actinomadura spiculosospora* nov. sp. K—4 (FERM P—6101) handelt.

13. Pharmazeutische Zusammensetzung, die eine wirksame Menge der Verbindung (I) und ein oder mehrere pharmakologisch unbedenkliche Vehikel enthält.

14. Pharmazeutische Zusammensetzung gemäß Anspruch 13, in der die Konfigurationen der beiden Aminosäurenteile L-förmig sind und der Teil 1-Amino-2-(4-hydroxyphenyl)ethylphosphonsäure gegenüber der Verbindung (I) eine negative spezifische Drehung hat.

15. Ein Mikroorganismus *Actinomycetes* K—26 (FERM P—5889, NRRL 12379).

16. Ein Mikroorganismus *Actinomadura spiculosospora* nov. sp. K—4 (FERM P—6101).

**Revendications**

1. Oligopeptides phosphorés représentés par la formule [I]:

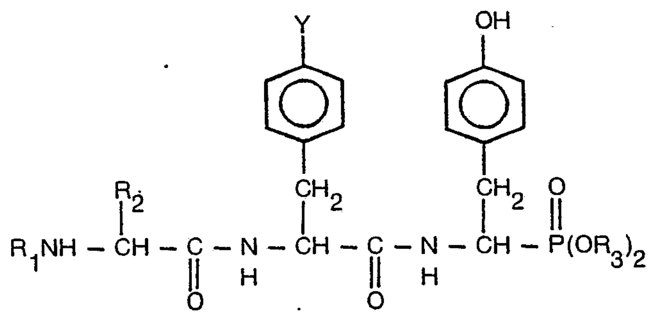

[I]

dans laquelle $R_1$ représente l'hydrogène, un groupe alkyle ayant 1 à 6 atomes de carbone, phénylalkyle non substitué ou substitué (dans lequel la portion alkyle possède 1 à 6 atomes de carbone et le substituant est choisi parmi les groupes halogéno, nitro et alcoxy ayant 1 à 3 atomes de carbone) ou $R_4CO$—, dans lequel $R_4$ représente l'hydrogène, un groupe alkyle ayant 1 à 6 atomes de carbone ou phénylalkyle non substitué ou substitué (dans lequel la portion alkyle possède 1 à 6 atomes de carbone et le substituant est choisi parmi les groupes halogéno, nitro et alcoxy ayant 1 à 3 atomes de carbone), $R_2$ représente un groupe alkyle ayant 1 à 6 atomes de carbone; $R_3$ représente l'hydrogène, un groupe alkyle ayant 1 à 6 atomes de carbone ou phénylalkyle non substitué ou substitué (dans lequel la portion alkyle possède 1 à 6 atomes de carbone et le substituant est choisi parmi les groupes halogéno, nitro et alcoxy ayant 1 à 3 atomes de carbone) et Y représente l'hydrogène ou le groupe hydroxyle.

2. Oligopeptides phosphorés selon la revendication 1, dans lesquels la configuration des radicaux dérivés des deux aminoacides se présente sous la forme L et le radical dérivé de l'acide 1-amino-2-(4-hydroxyphényl)éthylphosphonique a une rotation spécifique négative.

3. Oligopeptides phosphorés selon la revendication 2, dans lesquels $R_1$ est le groupe $R_4CO$—, $R_2$ est le groupe sec-butyle et Y est le groupe hydroxyle.

4. Oligopeptides phosphorés selon la revendication 3, dans lesquels $R_4$ est le groupe méthyle.

5. Oligopeptides phosphorés selon la revendication 2, dans lesquels $R_1$ est l'hydrogène, un groupe alkyle ou phénylalkyle substitué ou non substitué, $R_2$ est le groupe isopropyle et Y est l'hydrogène.

6. Oligopeptides phosphorés selon la revendication 5, dans lesquels $R_1$ est le groupe méthyle.

7. Procédé de préparation du composé [I] selon la revendication 1, qui comprend l'élimination du (des) groupe(s) protecteur(s) d'un composé représenté par la formule [V]:

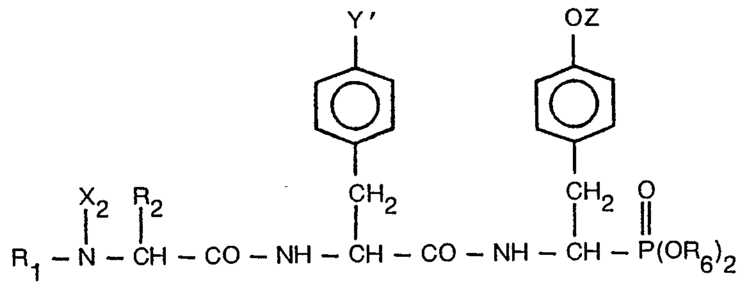

[V]

dans laquelle $X_2$ est l'hydrogène lorsque $R_1$ est le groupe $R_4CO$—, ou un groupe protecteur de fonction amine couramment utilisé dans la synthèse peptidique lorsque $R_1$ est l'hydrogène, un groupe alkyle ou phénylalkyle substitué ou non substitué; Y' représente l'hydrogène, un groupe hydroxyle ou OZ; Z représente un groupe protecteur de fonction hydroxyle couramment utilisé dans la synthèse peptidique; $R_6$ a la même signification que $R_3$, à l'exeption de l'hydrogène; et $R_1$, $R_2$, $R_3$ et $R_4$ ont les mêmes significations que celles définies dans la revendication 1 et, éventuellement, l'hydrolyse du composé résultant.

8. Procédé de préparation du K—26, à savoir l'acide N-(N-acétyl-L-isoleucyl-L-tyrosyl)-(−)-1-amino-2-(4-hydroxyphényl)éthylphosphonique, qui comprend la culture d'un microorganisme appartenant à *Actinomycetes* et ayant une capacité pour produire le K—26 dans un milieu de culture, la formation et l'accumulation du K—26 dans la liqueur de culture et la récupération du K—26 depuis cette liqueur de culture.

9. Procédé selon la revendication 8, dans lequel le microorganisme est *Actinomycetes* K—26 (FERM P—5889, NRRL 12379).

10. Procédé pour la préparation du K—4, à savoir l'acide N-(N-méthyl-L-valyl-L-phénylalanyl)-(−)-1-amino-2-(4-hydroxyphényl)éthylphosphonique, qui comprend la culture d'un microorganisme appartenant au gène *Actinomadura* et ayant une capacité pour produire le K—4 dans un milieu de culture, la formation et l'accumulation du K—4 dans la liqueur de culture et la récupération du K—4 depuis cette liqueur de culture.

11. Procédé selon la revendication 10, dans lequel le microorganisme appartient à l'espèce *Actinomadura spiculosospora*.

12. Procédé selon la revendication 11, dans lequel le microorganisme est la nouvelle espèce K—4 de *Actinomadura spiculosospora* (FERM P—6101).

13. Composition pharmaceutique qui comprend une quantité efficace de composé [I] selon la revendication 1 et un (des) excipient(s) pharmacologiquement acceptable(s).

14. Composition pharmaceutique selon la revendication 13, dans laquelle les configurations des deux radicaux dérivés des deux aminoacides se présentent sous la forme L et le radical dérivé de l'acide 1-amino-2-(4-hydroxyphényl)éthylphosphonique a une rotation spécifique négative.

15. Microorganisme *Actinomycetes* K—26 (FERM P—5889 NRRL 12379).

16. Microorganisme *Actinomadura spiculosospora* nouvelle espèce K—4 (FERM P—6101).

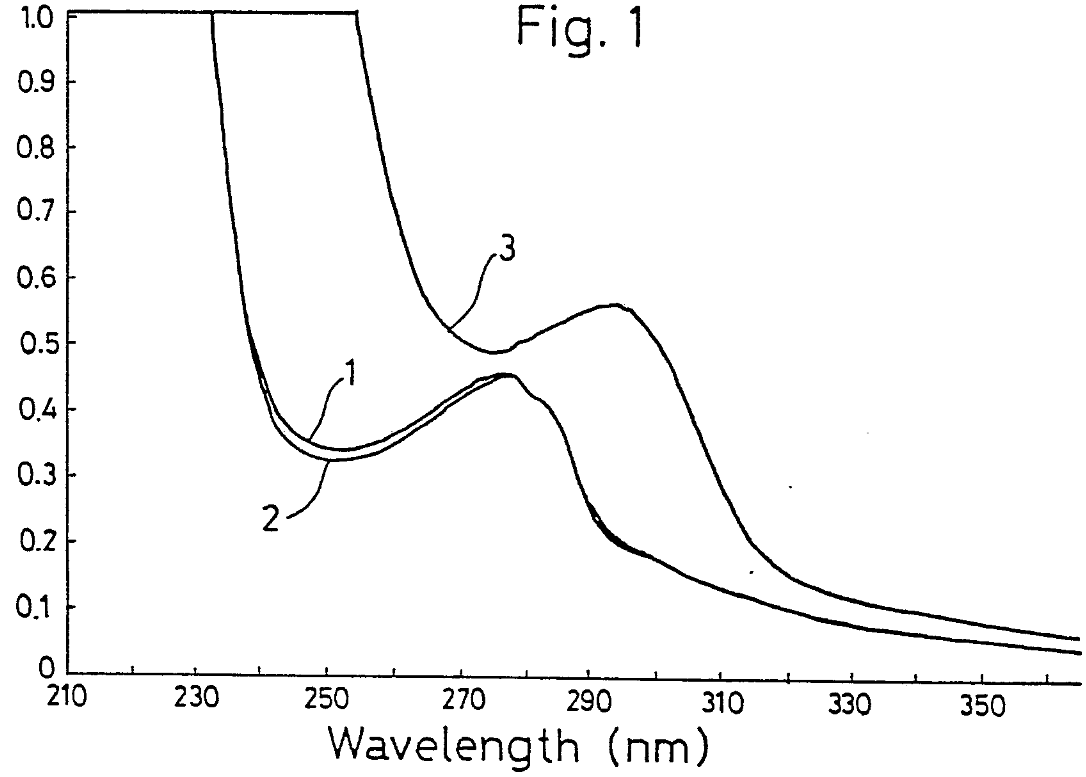
Fig. 1
1.0
0.9
0.8
0.7
0.6
0.5
0.4
0.3
0.2
0.1
0
210
230
250
270
290
310
330
350
Wavelength (nm)
1
2
3

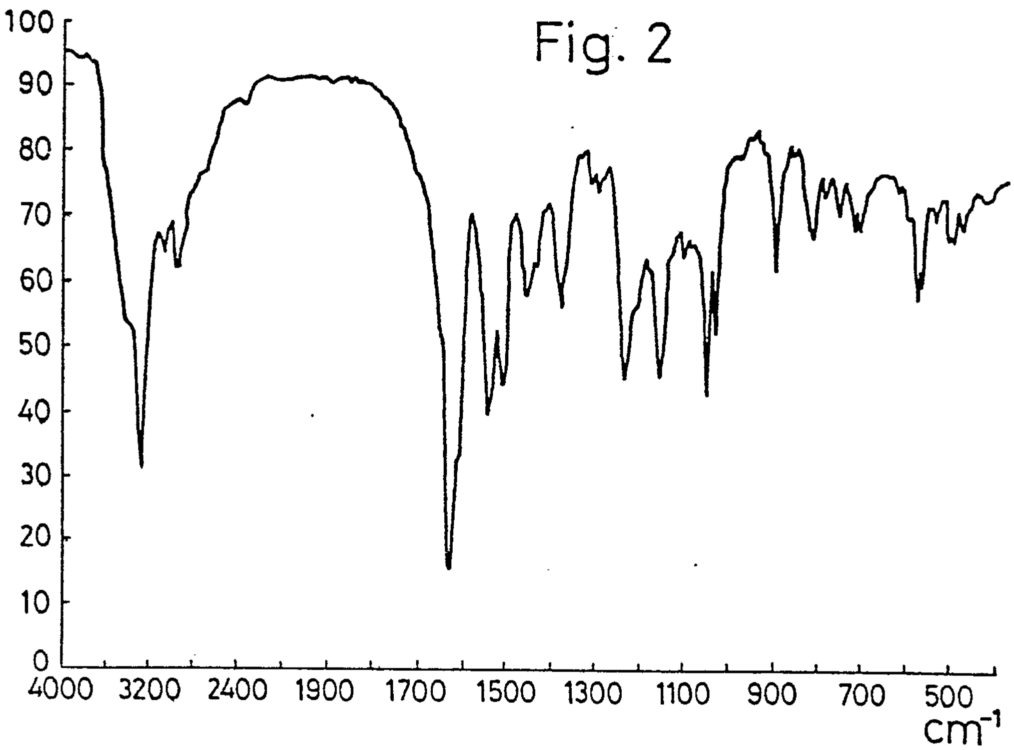
Fig. 2
100
90
80
70
60
50
40
30
20
10
0
4000
3200
2400
1900
1700
1500
1300
1100
900
700
500
cm-1

Fig. 3

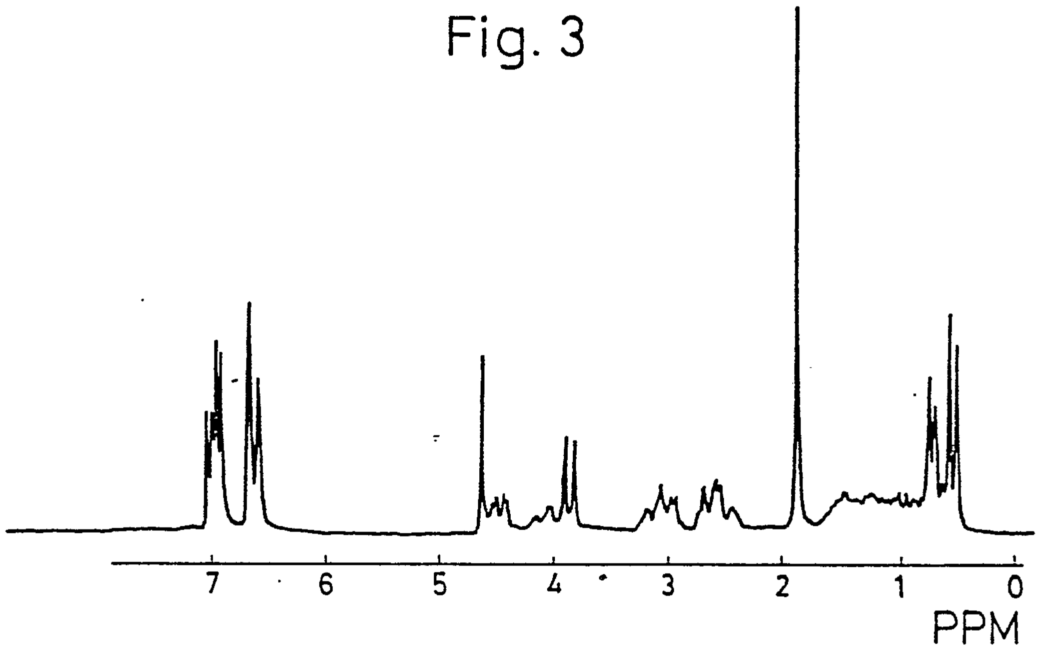


Fig. 4

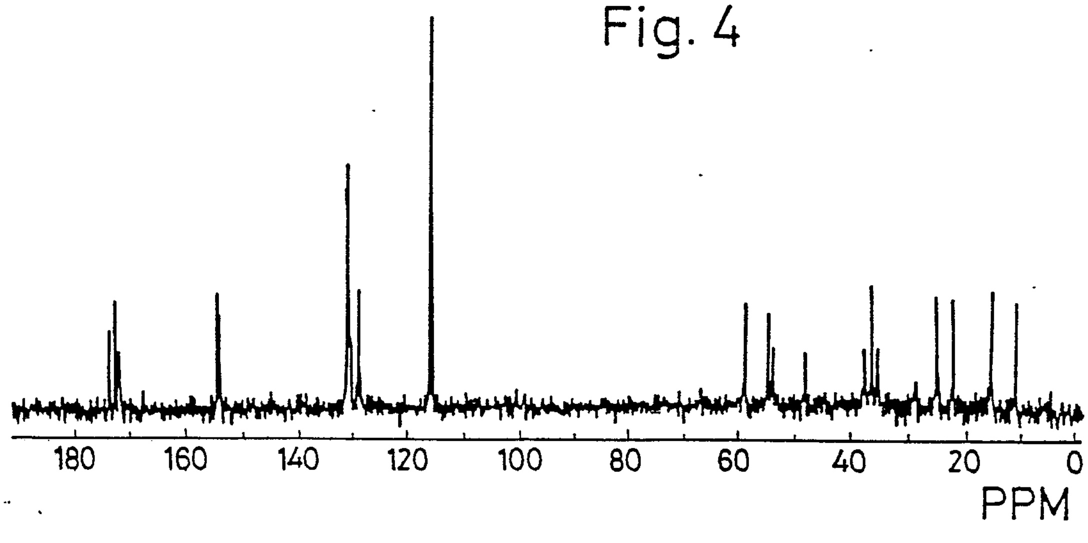

Fig. 5

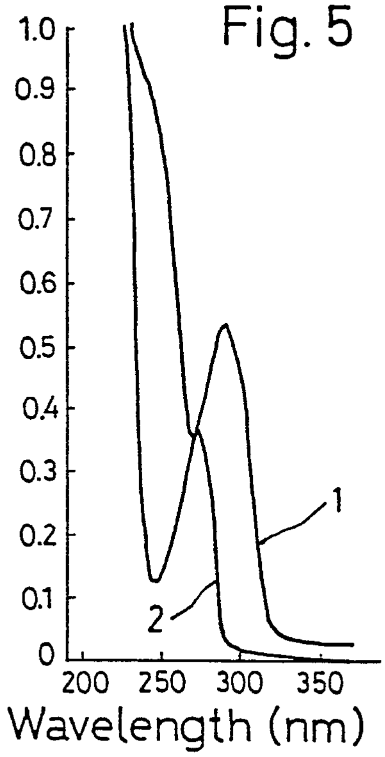
1.0
0.9
0.8
0.7
0.6
0.5
0.4
0.3
0.2
0.1
0
200
250
300
350
Wavelength (nm)
1
2

Fig. 6

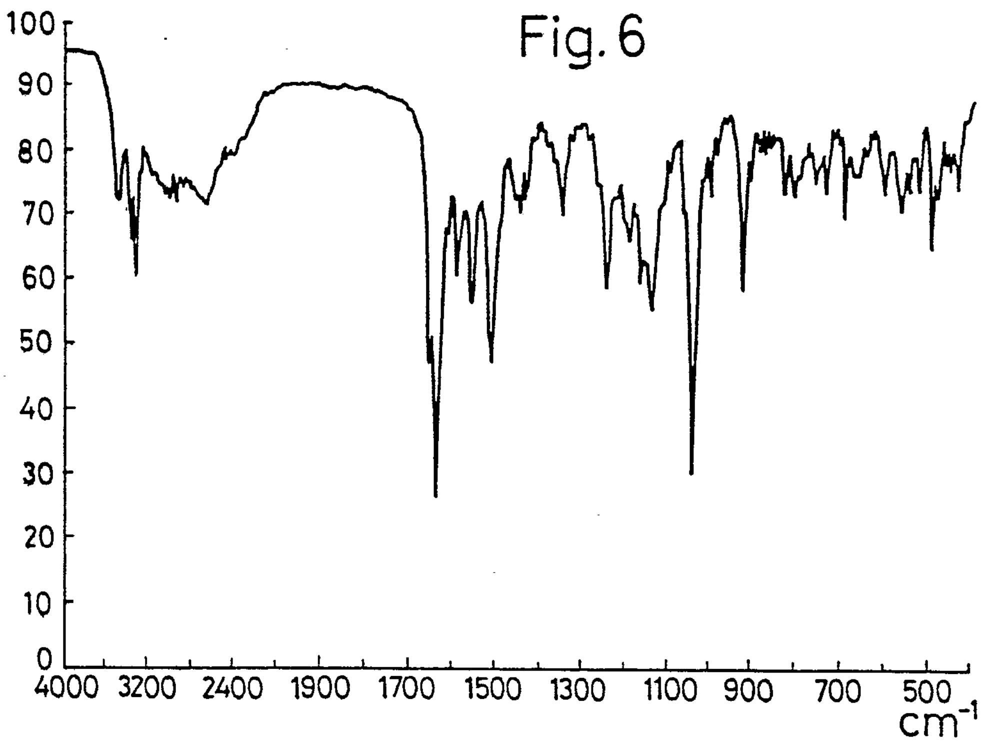
100
90
80
70
60
50
40
30
20
10
0
4000
3200
2400
1900
1700
1500
1300
1100
900
700
500
cm⁻¹

Fig. 7

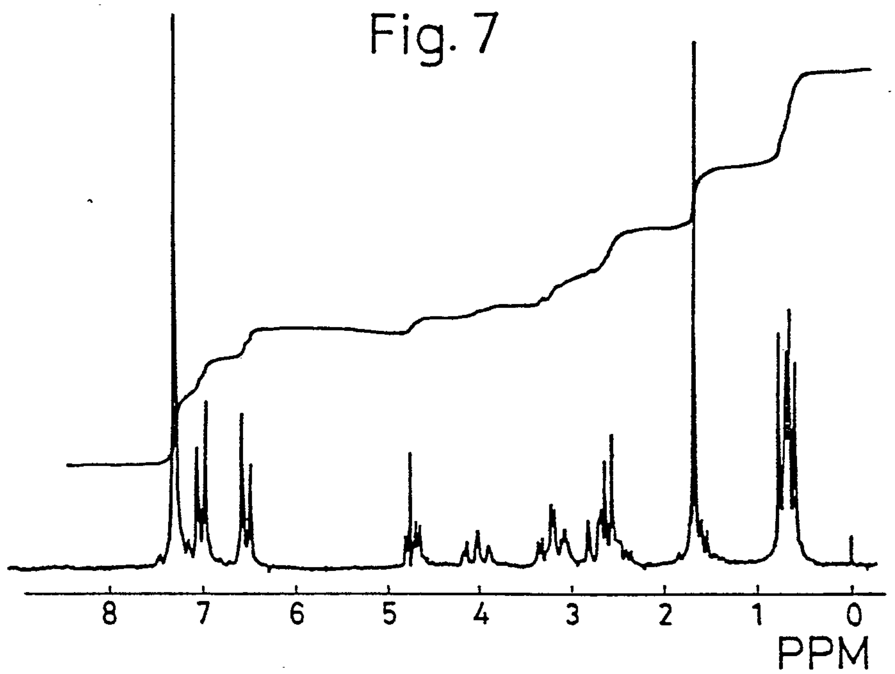


Fig. 8